# EUROPEAN PATENT APPLICATION

(11) **EP 4 327 736 A1**
(43) Date of publication of application: **28.02.2024**
(21) Application number: 21942824.0
(22) Date of filing: 24.12.2021
(51) Int. Cl.: A61B 5/055, A61B 6/03

(54) **ANIMAL COMPARTMENT AND ANIMAL IMAGING APPARATUS**

(30) Priority: 27.05.2021 CN 202110584695; 27.05.2021 CN 202121162307 U; 05.07.2021 CN 202121513677 U; 26.09.2021 CN 202122344136 U
(71) Applicant: Wuhan United Imaging Life Science Instrument Co., Ltd, Wuhan, Hubei 430206 (CN)
(72) Inventor: LEI, Hongxia, Wuhan, Hubei 430206 (CN); HONG, Tao, Wuhan, Hubei 430206 (CN); WU, Qi, Wuhan, Hubei 430206 (CN)
(74) Representative: Murgitroyd & Company
(86) International application number: PCT/CN2021/141270
(87) International publication number: WO 2022/247261

(57) **Abstract**

An animal compartment and an animal imaging apparatus. The animal compartment comprises a first compartment body (10) and a second compartment body (210), the first compartment body (10) being detachably connected to the second compartment body (210). The animal compartment is applied to the animal imaging apparatus, such that during scan imaging of an animal, only the second compartment body (210) needs to be replaced to meet the requirements of different scanning scenarios.

## Description

### TECHNICAL FIELD

The present application relates to the field of life science technology, and in particular to an animal cabin and an animal imaging device.

### BACKGROUND

Biologically relevant small animal models have become important tools for studying fundamental aspects of human system functions and dysfunctions. The understanding of human functioning has been greatly enhanced by observing changes at the levels of systems, organs, tissues, cells, and molecules in response to different physiological or environmental conditions and disease states under specified experimental conditions. Preclinical magnetic resonance imaging (MRI) is a particularly attractive imaging option for evaluating animal models, as it provides good spatial resolution without harmful radiation. It also has excellent contrast to differentiate between normal and pathological tissues. In addition to providing anatomical information, preclinical MRI can also perform functional assessments through, for example, visualization of flow quantification, tissue diffusion and perfusion, or blood oxygenation changes.

The existing animal cabin in the magnetic resonance environment is directly connected to the animal support bed. For different scanning scenarios, such as the need for magnetic resonance imaging of animals with various body sizes, the entire animal cabin needs to be replaced, which is a complicated operation.

### SUMMARY

Based on this, embodiments of the present application provide an animal cabin and an animal imaging device. For different scanning scenarios, such as the need for magnetic resonance imaging of animals with various body sizes, only a second capsule needs to be replaced, thereby making it easy to operate.

An embodiment of the present application provides an animal cabin, including a first capsule and a second capsule, and the first capsule is detachably connected to the second capsule.

When the above-mentioned animal cabin is used in the animal imaging device to scan and image animals, to accommodate different scanning scenarios, such as the need to scan and image animals of various body sizes, only the second capsule needs to be replaced, thereby making it easy to operate.

In an embodiment, the animal cabin further includes a first adjusting assembly. The first capsule and the second capsule are connected through the first adjusting assembly. The animal cabin further includes a rotating portion located between the first capsule and the second capsule. Under an adjustment of the first adjusting assembly, the second capsule is capable of rotating around the rotating portion relative to the first capsule to adjust an angle between the first capsule and the second capsule.

In an embodiment, the rotating portion includes a rotating convex portion and a rotating concave portion. The rotating convex portion is located at an end of the first capsule adjacent to the second capsule, the rotating concave portion is located at an end of the second capsule adjacent to the first capsule, and the rotating convex portion concave-convex fits with the rotating concave portion.

Alternatively, the rotating concave portion is located at an end of the first capsule adj acent to the second capsule, the rotating convex portion is located at an end of the second capsule adjacent to the first capsule, and the rotating convex portion concave-convex fits with the rotating concave portion.

In an embodiment, the rotating convex portion has a first arc surface thereon, and the rotating concave portion has a second arc surface therein, and the first arc surface and the second arc surface are attached to each other in accordance with the concave-convex fit of the rotating convex portion and the rotating concave portion.

In an embodiment, the first adjusting assembly includes an adjusting rod, the second capsule is provided with a first connection hole extending through the second capsule, the first capsule is provided with an adjustment hole, an end of the adjusting rod extends through the first connection hole and is capable of cooperating with the adjustment hole to adjust an angle of the second capsule relative to the first capsule.

In an embodiment, the adjusting rod includes a first threaded rod with a head, and the head abuts against the second capsule, the adjustment hole is a threaded hole, and the first threaded rod is in threaded fit with the adjustment hole.

In an embodiment, a side wall of the first capsule adjacent to the second capsule is provided with an abutting portion, a side wall of the second capsule adjacent to first capsule is provided with a receiving groove configured to accommodate the abutting portion, and when the first capsule is connected to the second capsule, the abutting portion abuts against a groove wall of the receiving groove.

In an embodiment, the second capsule is provided with a mounting hole at a side towards the first capsule, the mounting hole is in communication with the first connection hole. An elastic unit is provided in the mounting hole, and the elastic unit is capable of elastically abutting against the first capsule as adjustment of the adjusting rod.

In an embodiment, the animal cabin further includes a cover. The second capsule is provided with a chamber, the cover is detachably connected to the second capsule, and the cover is capable of covering and being fixed on the second capsule.

In an embodiment, the animal cabin further includes a locking member. The locking member is disposed on the cover and an end of the second capsule, the locking member is capable of locking the cover and the second capsule, thereby locking and fixing the cover body along an axis direction of the second capsule, and the cover body and another end of the second capsule body are engaged with each other.

In an embodiment, the second capsule includes a first part and a second part arranged sequentially in the axis direction of the second capsule, and the cover covers and detachably connected to the second part;

an end of the cover adjacent to the first part is provided with a positioning protrusion extending toward the first part, the first part is provided with a groove adapted to the positioning protrusion, the positioning protrusion extends into the groove, the groove has a groove wall, the locking member is inserted through the positioning protrusion and the groove wall to lock the second capsule and the cover.

In an embodiment, the second capsule includes a first part and a second part arranged sequentially in the axis direction of the second capsule, and the cover covers and detachably connected to the second part;
an end of the cover away from the first part is provided with a first buckle, an end of the second part away from the first part is provided with a second buckle, the first buckle and the second buckle are capable of being locked with each other;
along the axis direction of the second capsule, the first buckle and the second buckle are capable of separating from each other; and
along a direction perpendicular to the axis direction of the second capsule, the first buckle and the second buckle are locked with each other.

In an embodiment, the animal cabin further includes a first joint and a second joint respectively disposed at both ends of the second part. The first joint and the second joint are in communication with the chamber

In an embodiment, a top of the cover away from the second part is a planar structure.

In an embodiment, the animal cabin further includes an animal fixation device mounted in the second capsule and configured to fix an animal to be scanned.

In an embodiment, the animal fixation device includes:
a support base;
a tooth positioning portion, mounted on the support base and configured to position teeth of an animal; and
a respiratory mask, configured to provide an air channel for the animal, wherein the respiratory mask is fixed on the tooth positioning portion.

In an embodiment, the respiratory mask includes a mask mounting portion and an air chamber, the mask mounting portion is provided with a mounting groove in communication with the air chamber, and the tooth positioning portion is inserted into the mounting groove.

In an embodiment, the mounting groove has a first inner wall and a second inner wall opposite to each other, the tooth positioning portion is sandwiched between the first inner wall and the second inner wall, and along a direction in which the tooth positioning portion is inserted into the mounting groove, a distance between the first inner wall and the second inner wall gradually decreases.

In an embodiment, the tooth positioning portion is provided with a tooth hole, the tooth positioning portion is provided with a ventilation hole, the mask mounting portion is provided with an exhaust channel, and a position of the exhaust channel corresponds to a position of the ventilation hole.

In an embodiment, the animal fixation device further includes a frontal bone positioning portion mounted on the support base, and the frontal bone positioning portion is configured to press the frontal bone of the animal in a direction toward the tooth positioning portion.

In an embodiment, the animal fixation device further includes a second adjusting assembly, the second adjusting assembly includes a limiting member and a second elastic member. The limiting member is adjustably connected to the tooth positioning portion and is capable of adjusting a position thereof in a direction toward or away from the tooth positioning portion. The second elastic member is configured to exert an elastic force on the frontal bone positioning portion in a direction away from the tooth positioning portion, such that an end of the frontal bone positioning portion away from the tooth positioning portion abuts against limiting member.

In an embodiment, the limiting member is a threaded connection member and includes a connection head portion and a second threaded rod fixed to the connection head portion, the connection head portion is located on a side of the frontal bone positioning portion away from the tooth positioning portion, the second threaded rod extends through the frontal bone positioning portion and is threadedly connected to the tooth positioning portion, the second elastic member is sleeved on the second threaded rod and is located between the frontal bone positioning portion and the tooth positioning portion;
an end of the frontal bone positioning portion is rotatably connected to the tooth positioning portion, and when the threaded connection member rotates, the threaded connection member or the second elastic member is capable of driving the frontal bone positioning portion to rotate relative to the tooth positioning portion.

In an embodiment, the animal fixation device includes the support base, and two ear rods opposite to each other, the ear rods are mounted on the support base, each ear rod is provided with an auditory stimulation input hole; and an end of each ear rod has a rough surface or an earmuff is provided on the ear rod.

An embodiment of the present application further provides an animal imaging device, including any one of the above-mentioned animal cabin and a scanning device. The scanning device has a scanning chamber, the animal cabin is capable of extending into the scanning chamber, and he scanning device is configured to scan and form an image of an animal in the animal cabin.

When the animal cabin of the above-mentioned animal imaging device is used in the animal imaging device to scan and image animals, according to different scanning scene requirements, for example, when it is necessary to scan and form an image of animals of different body sizes, only the second capsule needs to be replaced, thereby making it easy to operate.

### BRIEF DESCRIPTION OF THE DRAWINGS

FIG. 1 is a schematic structural view of an animal cabin according to some embodiments.
FIG. 2 is a schematic view of the animal cabin in FIG. 1 from another perspective.
FIG. 3 is a schematic structural view of a first capsule of the animal cabin in FIG. 1.
FIG. 4 is a schematic structural view of a second capsule of the animal cabin in FIG. 1.
FIG. 5 is a schematic structural view of a first adjusting assembly of the animal cabin in FIG. 1.
FIG. 6 is a schematic structural view of the animal cabin in FIG. 1 before leveling.
FIG. 7 is a schematic structural view of the animal cabin in FIG. 1 in an extreme position after adjustment.
FIG. 8 is a schematic view showing a connection relationship between the second capsule and a cover of the animal cabin in FIG. 1.
FIG. 9 is a schematic structural view of the second capsule in FIG. 8.
FIG. 10 is a schematic structural view of the cover in FIG. 8.
FIG. 11 is a partial cross-sectional schematic structural view of FIG. 8.
FIG. 12 is an enlarged schematic structural view of A of FIG. 11.
FIG. 13 is a schematic view of an overall structure of an animal fixation device according to some embodiments.
FIG. 14 is a schematic structural view of a support base in FIG. 13.
FIG. 15 is a schematic structural view of a tooth positioning portion in FIG. 13.
FIG. 16 is a schematic view showing a connection relationship between a frontal bone positioning portion and the tooth positioning portion in FIG. 13.
FIG. 17 is a schematic structural view of a respiratory mask in FIG. 13.
FIG. 18 is a schematic structural view of a base in FIG. 13.
FIG. 19 is a schematic view showing a connection relationship between a mounting seat and the base in FIG. 13.
FIG. 20A is a schematic structural view of an ear positioning portion in FIG. 13.
FIG. 20B is a schematic structural view of an ear positioning portion according to some other embodiments.
FIG. 20C is a schematic structural view of an ear positioning portion according to yet some other embodiments.

### Description of reference signs:

1, animal cabin;
10, first capsule; 11, adjustment hole; 12, protrusion; 13, recessed portion; 14, abutting portion;
210, second capsule; 21, first connection hole; 22, mounting hole; 23, protruding portion; 24, receiving groove; 211, chamber; 212, first part; 2121, groove; 2121a, groove wall; 2122, wire passing groove; 2123, first joint; 2124, accommodating groove; 2124a, first wall; 2124b, second wall; 2125, fixing portion; 213, second part; 2131, second buckle; 2131a, second inserting portion; 2131b, second recessed portion; 2132, second joint;
220, cover; 221, positioning protrusion; 2211, second connection hole; 222, first buckle; 2221, first inserting portion; 2222, first recessed portion; 223, wire port; 224, planar structure;
230, locking member;
30, first adjusting assembly; 31, adjusting rod; 311, first threaded rod; 312, head; 32, elastic unit; 321, first elastic member; 322, guide post;
40, rotating portion; 41, rotating convex portion; 411, first arc surface; 42, rotating concave portion; 421, second arc surface;
100, animal fixation device;
110, base; 111, bottom plate; 109, base mounting hole; 112, limiting plate; 112a, coil mounting hole; 113; second mounting protrusion; body placement slot 106;
120, ear positioning portion; 121, first threaded connection member; 122a, tooth; 122b, tooth groove; 123, tapering segment; 108, auditory stimulation input hole;
130, tooth positioning portion; 101, tooth hole; 102, ventilation hole; 131, first mounting shaft; 132, first mounting post; 133, rotating shaft;
140, frontal bone positioning portion; 141, positioning portion body; 142, bending portion; 142a, arc-shaped concave surface;
150, respiratory mask; 151, mask body; 103, air chamber; 1031, opening of the air chamber 103; 152, mask mounting portion; 104, mounting groove; 1041, first inner wall; 1042, second inner wall; 1042a, first part; 1042b, second part; 105, exhaust channel; 153, air inlet pipe joint; 154, exhaust pipe joint;
160, support base; 161, support body; 162, first support arm; 163, second support arm; 164, first mounting protrusion; 164a, first mounting hole; 165, second threaded connection member; 166, second mounting shaft; 167, third threaded connection member;
170, second adjusting assembly; 171, limiting member; 1711, head portion; 1712, second threaded rod; 172, second elastic member;
180, mounting seat; 181, mounting body; 182, third mounting shaft; 183, fourth threaded connection member;
190, height adjustment pad.

### DETAILED DESCRIPTION OF THE EMBODIMENTS

In order to make the above objects, features and advantages of the present application clear and easier to understand, the specific embodiments of the present application are described in detail below in combination with the accompanying drawings. Many specific details are set forth in the following description to facilitate a full understanding of the present application. However, the present application can be implemented in many ways different from those described herein, and those skilled in the art can make similar improvements without departing from the connotation of the present application. Therefore, the present application is not limited by the specific embodiments disclosed below.

In the description of the present application, it should be understood that the terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential direction" are based on the azimuth or position relationship shown in the attached drawings, which are only for the convenience of describing the present application and simplifying the description, rather than indicating or implying that the device or element must have a specific azimuth, be constructed and operated in a specific azimuth, so such terms cannot be understood as a limitation of the present application.

In addition, the terms "first" and "second" are only used for descriptive purposes and cannot be understood as indicating or implying relative importance or implicitly indicating the number of indicated technical features. Thus, the features defined with "first" and "second" may explicitly or implicitly include at least one of the features. In the description of the present application, "a plurality of' means at least two, such as two, three, etc., unless otherwise expressly and specifically defined.

In the present application, unless otherwise expressly specified and limited, the terms "mount", "connect", "contact", "fix" and other terms should be understood in a broad sense, for example, they can be fixed connections, detachable connections, or integrated. They can be mechanical connection or electrical connection. They can be directly connected or indirectly connected through an intermediate medium. They can be the connection within two elements or the interaction relationship between two elements, unless otherwise expressly limited. For those skilled in the art, the specific meaning of the above terms in the present application can be understood according to the specific situation.

In the present application, unless otherwise expressly specified and limited, the first feature "above" or "below" the second feature may be in direct contact with the first and second features, or the first and second features may be in indirect contact through an intermediate medium. Moreover, the first feature is "above" the second feature, but the first feature is directly above or diagonally above the second feature, or it only means that the horizontal height of the first feature is higher than the second feature. The first feature is "below" of the second feature, which can mean that the first feature is directly below or obliquely below the second feature, or simply that the horizontal height of the first feature is less than that of the second feature.

It should be noted that when an element is called "fixed to" or "provided on" another element, it can be directly on another element or there can be a centered element. When an element is considered to be "connected" to another element, it can be directly connected to another element or there may be intermediate elements at the same time. The terms "vertical", "horizontal", "up", "down", "left", "right" and similar expressions used herein are for the purpose of illustration only and do not represent the only embodiment.

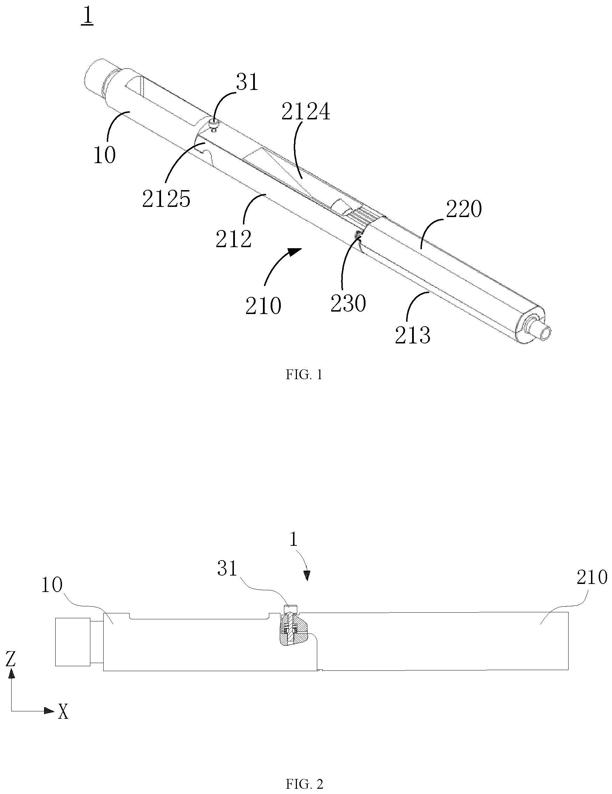

In some embodiments, referring to FIGs. 1 and 2, an animal cabin 1 is provided, and the animal cabin 1 is employed in an animal imaging device, especially in an animal imaging device combining magnetic resonance with other imaging modes, such as MR, PET-MR, and other imaging devices. The animal cabin 1 is mainly configured to accommodate mice, rats, rabbits and other experimental animals to be scanned, as well as other related devices.

Some embodiments of the present application provide an animal cabin 1, which can be extended into a scanning chamber of a scanning device of an animal imaging device. The animal cabin 1 includes a first capsule 10 and a second capsule 210. The first capsule 10 is detachably connected to the second capsule 210. The first capsule 10 is configured to connect to an animal support bed (not shown). The first capsule 10 can be fixed on the animal support bed. The first capsule 10 is detachably connected to the animal support bed, or the first capsule 10 and the animal support bed are integrally formed. Taking the animal cabin used in a MR animal imaging device as an example to illustrate, the first capsule 10 can be configured to accommodate water pipe joints, light environment components, and other components, and the length of the first capsule 10 is associated with a feed stroke, a magnet size, and a central position of the animal support bed. The second capsule 210 is configured to accommodate and fix an animal to be scanned. The second capsule 210 is provided with an animal fixation accessory, other functional accessories for experiments, etc.

Since the first capsule 10 is detachably connected to the second capsule 210, the second capsule 210 can be detached from the first capsule 10. For different scanning scenarios, such as the need to scan and image animals of different body sizes, in the case that the second capsule 210 currently connected to the first capsule 10 is not suitable for the body size of the animal to be scanned, the current second capsule 210 connected to the first capsule 10 can be disassembled, and then the second capsule 210 adapted to the body size of the animal to be scanned is connected to the first capsule 10, so that the animal to be scanned can be placed in the second capsule 210. It can be seen that when the above-mentioned animal cabin 1 is used in the animal imaging device to scan and image animals, according to different scanning scenarios, such as the need to scan and image animals of different body sizes, only the second capsule 210 needs to be replaced, thereby making it easy to operate.

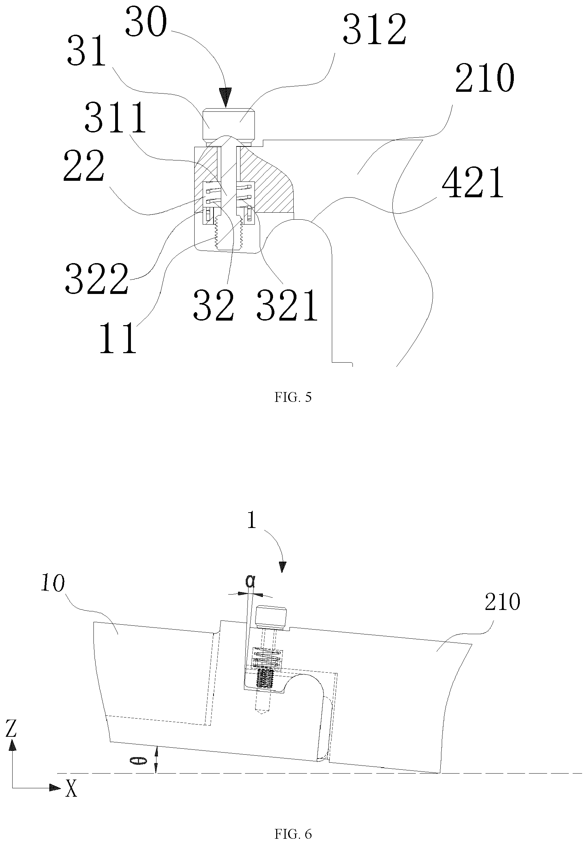

Referring to FIG. 5, in some embodiments, the animal cabin 1 further includes a first adjusting assembly 30. The first capsule 10 and the second capsule 210 are connected to each other through the first adjusting assembly 30. The animal cabin 1 further includes a rotating portion 40, which is disposed between the first capsule 10 and the second capsule 210. In addition, under an adjustment of the first adjusting assembly 30, the second capsule 210 can rotate around the rotating portion 40 relative to the first capsule 10 to adjust an angle between the first capsule 10 and the second capsule 210.

Due to the influence of gravity and processing and installation errors, the animal cabin 1 may sag at a certain angle. In some embodiments of the present application, the rotational angle between the first capsule 10 and the second capsule 210 can be in a narrow range. The second capsule 210 can rotate relative to the first capsule 10 within a range of 5 degrees. An axis of the second capsule 210 can be adjusted to a horizontal state by rotating the second capsule 210 by a small angle.

By disposing the rotating portion 40 between the first capsule 10 and the second capsule 210, when the animal cabin 1 tilts, the second capsule 210 can rotate relative to the first capsule 10 through the rotating portion 40 under the adjustment of the first adjusting assembly 30, realizing that a distal end of the animal bin 1 (the end at the second capsule) is in a horizontal state, which facilitate the animal cabin 1 to horizontally enter the scanning chamber of the animal imaging device.

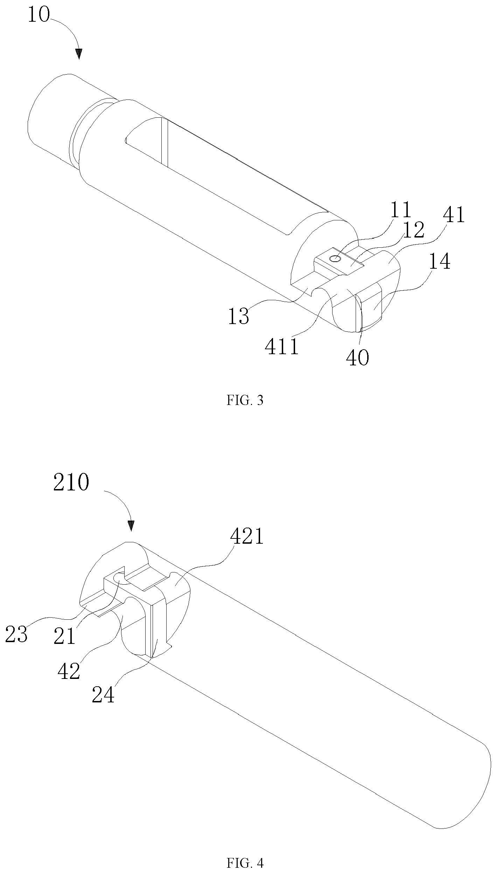

As shown in FIGs. 3 and 4, the first capsule 10 and the second capsule 210 are substantially cylindrical with an accommodating space therein.

In some embodiments, referring to FIG. 2, a direction extending along the axis of the animal cabin 1 in the horizontal state is defined as the X direction, that is, the X direction is located on the horizontal plane. Correspondingly, a vertical direction perpendicular to the axis of the animal cabin 1 is the Z direction, that is, the Z direction is perpendicular to the horizontal plane. It should be understood that the axis direction of the animal cabin 1 may be the length direction of the animal cabin.

In some embodiments, the first capsule 10 and the second capsule 210 are arranged and connected along the axis direction (the X direction in FIG. 2); that is, the first capsule 10 and the second capsule 210 connected to each other at their ends. When the first capsule 10 and the second capsule 210 are not tilted, and both parallel to the horizontal plane, the axes of the first capsule 10 and the second capsule 210 coincide. In some embodiments, the axis direction of the first capsule 10 and the axis direction of the second capsule 210 may be the length direction of the animal cabin.

Further, the first capsule 10 is provided with a recessed portion 13 recessed on a side of the rotating portion 40 away from the second capsule 210. Correspondingly, the second capsule 210 is provided with a protruding portion 23 protruding from a side of the rotating portion 40 adjacent to the first capsule 10. The protruding portion 23 and the recessed portion 13 can cooperate with each other, thereby restricting the first capsule 10 and the second capsule 210 from being separated from each other along the length direction of the animal cabin 1, thereby enhancing the connection strength between the first capsule 10 and the second capsule 210.

Certainly, in some embodiments, the protruding portion 23 can be alternatively disposed on the first capsule 10, and the recessed portion 13 can be disposed on the second capsule 210, as long as the first chamber 10 and the second chamber 210 can be restricted from being separated from each other along their own axial directions, and is not limited herein.

Further, the rotating portion 40 includes a rotating convex portion 41 and a rotating concave portion 42. The rotating convex portion 41 is located at an end of the recessed portion 13 adjacent to the second capsule 210, the rotating concave portion 42 is located at an end of the protruding portion 23 away from the first capsule 10, and the rotating convex portion 41 can concave-convex fit with the rotating concave portion 42. The rotating convex portion 41 protrudes upward relative to the recessed portion 13, and the rotating concave portion 42 is recessed upward relative to the protruding portion 23. It should be understood that the term "upward" refers to a vertical upward direction perpendicular to the axis directions of the first capsule 10 and the second capsule 210 when the first capsule 10 and the second capsule 210 are mounted horizontally.

It should be noted that protrusion and recess are two relative concepts. That is, in the case that the rotating convex portion 41 disposed on the first capsule 10 protrudes toward a direction adjacent to the second capsule 210, the recessed portion 13 on the first capsule 10 is a recess formed due to the protrusion of the rotating convex portion 41 relative to it. Similarly, the rotating concave portion 42 and the protruding portion 23 on the second capsule 210 also have the same relative concept.

Certainly, in some embodiments, the recessed portion 13 may be a groove further formed in the recess formed due to the protrusion of the rotating convex portion 41. The protruding portion 23 may be a protrusion further formed on the protrusion formed due to the recess of the rotating concave portion 42, which is not limited herein.

The rotating convex portion 41 is not limited to being disposed on the first capsule 10, and the rotating concave portion 42 is not limited to being disposed on the second capsule 210. It should be noted that the rotating convex portion 41 and the recessed portion 13 are both arranged on first capsule 10 or the second capsule 210, and the rotating concave portion 42 and the protruding portion 23 are both arranged on the first capsule 10 or the second capsule 210, so that the concave-convex fit between the first capsule 10 and the second capsule 210 can be achieved.

In some embodiments, the rotating convex portion 41 has a first arc surface 411 thereon, and the rotating concave portion 42 has a second arc surface 421 therein. The central axes of the first arc surface 411 and the second arc surface 421 are along the transverse direction of the animal cabin, that is, the direction perpendicular to the X direction and the Z direction (the XZ plane). When the concave-convex fit is formed between the rotation convex portion 41 and the rotation concave portion 42, the first arc surface 411 and the second arc surface 421 are in close contact with each other. In this case, the central axes of the first arc surface 411 and the second arc surface 421 substantially coincide. The second capsule 210 can rotate relative to the first capsule 10 with the central axes of the first arc surface 411 and the second arc surface 421 as the rotation axis, which enhances the rotation performance of the second capsule 210 relative to the first capsule 10.

Certainly, the realization of the relative rotation between the first capsule 10 and the second capsule 210 is not limited to providing the rotating convex portion 41 and the rotating concave portion 42 that are in concave-convex fit with each other. In some embodiments, the rotating portion 40 includes a rotating shaft. The rotating shaft is a separately provided component. Through holes for the rotating shaft to extend through are respectively provided in the first capsule 10 and the second capsule 210. The rotating shaft extends through the through holes of the first capsule 10 and the second capsule 210, so that not only the first capsule 10 can be connected to the second capsule 210, but also the second capsule 210 can rotate relative to the first capsule 10.

As shown in FIG. 5, the first adjusting assembly 30 includes an adjusting rod 31. The second capsule 210 is provided with a first connection hole 21 penetrating along the Z direction. The first capsule 10 is provided with an adjustment hole 11 corresponding to the first connection hole 21. An end of the adjusting rod 31 extends through the first connection hole 21, and can cooperate with the adjustment hole 11. The second capsule 210 and the first capsule 10 are in concave-convex fit through the rotating convex portion 41 and the rotating concave portion 42, and the recessed portion 13 and the protruding portion 23. In this case, the second capsule 210 overlaps the first capsule 10. An end of the adjusting rod 31 passes through the first connection hole 21 of the second capsule 210 and extends into the adjustment hole 11 of the first capsule 10, thereby realizing the connection between the first capsule 10 and the second capsule 210.

Specifically, the adjusting rod 31 includes a first threaded rod 311 with a head 312. The head 312 abuts against the second capsule 210. The adjustment hole 11 is a threaded hole. The first threaded rod 311 is in threaded fit with the adjustment hole 11. When the adjusting rod 31 is inserted through the first connection hole 21 into the adjustment hole 11 of the first capsule 10, the adjusting rod 31 is rotated, so that the threaded fit between the first threaded rod 311 and the adjustment hole 11 can convert rotation of the first threaded rod 311 within the adjustment hole 11 into movement of the first threaded rod 311 in the vertical direction.

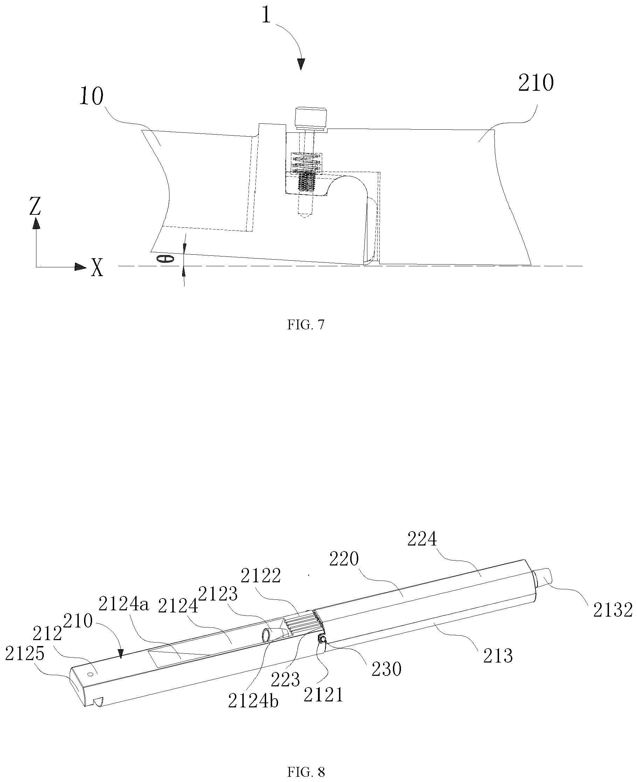

As shown in FIGs. 6 to 7, when the animal cabin 1 as a whole is tilted relative to the animal support bed, the second capsule 210 and the first capsule 10 as a whole are tilted downward by a certain angle in the Z direction, that is, the axes of the second capsule 210 and the first capsule 10 form an angle θ with the X direction. In this case, the adjusting rod 31 can be rotated to level the second capsule 210 to the X direction. During the rotation of the adjusting rod 31, the adjusting rod 31 will also move downward along its own axis in the Z direction. In this case, the adjusting rod 31 will exert an abutment force in the direction of movement of the adjusting rod 31 on the second capsule 210. Under the action of the abutment force, the angle between the second capsule 210 and the horizontal plane gradually reduces until the second capsule 210is adjusted to be horizontal, and the axis of second capsule 210 is parallel to the X direction (as shown in FIG. 7).

In an embodiment, a side wall above the recessed portion 13 of the first capsule 10 is an inclined surface. When the first capsule 10 and the second capsule 210 are mounted horizontally, the inclined surface of the side wall above the recessed portion 13 and an end side of the second capsule 210 form an initial angle α (referring to FIG. 6). When the first capsule 10 and the second capsule 210 are tilted, during the process of adjusting the second capsule 210, the adjusting rod 31 is rotated. The adjusting rod 31 moves downward along its own axis in the Z direction, and the angle between the inclined surface of the side wall above the recessed portion 13 of the first capsule 10 and the end side of the second capsule 210 gradually reduces, so that the second capsule 210 can be gradually leveled. The extreme state of the relative position of the first capsule 10 and the second capsule 210 can be referred to FIG. 7. In this case, the inclined surface of the side wall above the recessed portion 13 of the first capsule 10 is completely in contact with the end side of the second capsule 210.

Further, a side wall of the first capsule 10 adjacent to the second capsule 210 is provided with an abutting portion 14, and a side wall of the second capsule 210 adjacent to first capsule 10 is provided with a receiving groove 24 for accommodating the abutting portion 14. When the first capsule 10 is connected to the second capsule 210, the abutting portion 14 can abut against a groove wall of the receiving groove 24.

It should be noted that when the first capsule 10 is connected to the second capsule 210, the abutting portion 14 can abut against the groove wall of the receiving groove 24, so that the abutment force between the abutting portion 14 and the groove wall of the receiving groove 24 provides a fulcrum for the second capsule 210 to rotate relative to the first capsule 10, thereby making it easier for the second capsule 210 to be leveled.

Specifically, the abutting portion 14 protrudes in a direction toward the second capsule 210 along the axial direction of the first capsule 10, and the receiving groove 24 is recessed in a direction toward the second capsule 210 along the axial direction of the second capsule 210. The abutting portion 14 can provide special support for the second capsule 210, and the concave-convex fit of the abutting portion and the receiving groove can further limit and position the animal cabin in the transverse direction.

Further, the second capsule 210 is provided with a mounting hole 22 in communication with the first connection hole 21. The mounting hole 22 is disposed below the first connection hole 21 and in communication with the connection hole. An elastic unit 32 is disposed in the mounting hole 22. The elastic unit 32 partially protrudes from the mounting hole 22. The elastic unit 32 can elastically abut against the first capsule 10 as adjustment of the adjusting rod 31.

It should be noted that since the elastic unit 32 protrudes from the mounting hole 22, when the adjusting rod 31 is rotated, the elastic unit 32 abuts against the first capsule 10 downward along the Z direction. In this case, the elastic unit 32 abuts against the first capsule 10 under the action of the abutment force, causing the elastic unit 32 to contract in the mounting hole 22. The elastic unit 32 exerts an abutment force in an opposite direction on the second capsule 210 due to the restoring force, so that the second capsule 210 is stably connected to the first capsule 10 under the cooperative action of the elastic unit 32 and the adjusting rod 31, ensuring the stability of the leveled second capsule 210.

Specifically, the elastic unit 32 includes a first elastic member 321 and a guide post 322 that are connected to each other. An end of the first elastic member 321 abuts against a hole wall of the mounting hole 22, and another end of the first elastic member 321 is connected to the guide post 322. The guide post 322 partially protrudes out from the mounting hole 22, and the guide post 322 is in contact with the hole wall of the mounting hole 22 and can move linearly along the hole wall of the mounting hole 22 driven by the first elastic member 321. The guide post 322 is configured to prevent the first elastic member 321 from tilting left or right during the expansion and contraction process.

When the adjusting rod 31 is rotated and the second capsule 210 moves downward along the Z direction, the guide post 322 also abuts against the first capsule 10 at this time. The guide post 322 is withdrawn in the mounting hole 22 under the elastic force of the first elastic member 321. The first elastic member 321 exerts a force on the guide post 322 against the first capsule 10 due to the restoring force, thus further enhancing the connection strength between the first capsule 10 and the second capsule 210.

It should be noted that the elastic unit 32 can instead only include the first elastic member 321, and the first elastic member 321 directly abuts against the first capsule 10, and as long as an abutment force can be exerted on the first capsule 10 through the elastic force, the specific arrangement manner of the elastic unit 32 is not limited herein.

In some embodiments, the first elastic member 321 is a spring. Certainly, in other embodiments, the first elastic member 321 can also be other elastic structures, which are not limited herein.

Further, the first capsule 10 is provided with a protrusion 12 protruding in a direction toward the second capsule 210. The adjustment hole 11 is opened in the protrusion 12, and the inner wall of the adjustment hole 11 has threads. An end of the first threaded rod 311 away from the head 312 extends into the adjustment hole 11 and is in threaded connection with the adjustment hole 11.

Specifically, the protrusion 12 is disposed on the recessed portion 13, and the guide post 322 can abut against the protrusion 12 with the concave-convex fit between the protruding portion 23 and the recessed portion 13. Since the protrusion 12 protrudes upward along the Z direction, the abutment force of the guide post 322 against the first capsule 10 is increased on the basis of the guide post 322 abutting against the first capsule 10.

The animal cabin 1 provided by the embodiment of the present application is includes the rotating portion 40 between the first capsule 10 and the second capsule 210, so that when the animal cabin 1 tilts, the second capsule 210 can rotate relative to the first capsule 10 through the rotating portion 40 under the adjustment of the first adjusting assembly 30, thereby achieving leveling of the animal cabin 1 and facilitating the horizontal entry of the animal cabin 1 into the magnet.

Referring to FIG. 8, in some embodiments, the animal cabin 1 further includes a cover 220. The cover 220 is configured to close the space for accommodating the animal to be scanned after placing the animal to be scanned in the second capsule 210. The second capsule 210 has a chamber 211, which is the space surrounded by the cover 220 and a second part 213. The chamber 211 is configured to receive the animal to be tested, animal fixation devices, wires, and other experiment-related components. The cover 220 is detachably connected to the second capsule 210, and the cover 220 can cover and be fixed to the second capsule 210 to seal the chamber 211.

In the present embodiment, on the one hand, the cover 220 is fixed to the second capsule 210, and on the other hand, the cover 220 is detachably connected to the second capsule 210, so that animals/objects can be conveniently placed in or taken out from the chamber 211.

As shown in FIG. 8, the second capsule 210 includes a first part 212 and the second part 213 that are fixed to each other. The cover 220 covers and is detachably connected to the second part 213. The first part 212 and the second part 213 are both connected to each other at their end positions. In other embodiments, the first part 212 and the second part 213 may also be connected to each other at their side positions or overlap each other, which is not limited herein. The second part 213 is configured to accommodate the animal to be scanned, and the first part 212 is configured to accommodate cables, pipes, and other devices connected to the interior of the second part 213.

In some embodiments, both the first part 212 and the second part 213 have a cylindrical structure. Certainly, in other embodiments, the first part 212 and the second part 213 may also have other shapes such as a cube, a cuboid, or the like.

In some embodiments, the first part 212 and the second part 213 are integrally formed using non-magnetic materials, so as to enhance the connection performance and structural strength between the first part 212 and the second part 213.

In some embodiments, the first part 212 is detachably connected to the second part 213. The first part 212 can be detachably connected to the second part 213 through a connecting member. The second part 213 can be disassembled and replaced with other second parts 213 and other accessories connected to the second part 213, such as coils and animal fixing components, to facilitate coil replacement and adapt to different coil components. Different animal fixing components, such as ear rods, respiratory masks and tooth positioning portions, etc., can also be fixed to the second part 213, which can adapt to animals of different sizes and are suitable for a variety of application scenarios.

In some embodiments, the connecting member may include a positioning pin disposed on the first part 212, and the second portion 213 is provided with a positioning hole matching the positioning pin at a position corresponding to the positioning pin. The positioning pin and the positioning hole cooperate with each other to realize the positioning and relative fixation of the first portion 212 and the second portion 213. Specifically, the connecting member may further include a plurality of connecting screws, through which the first part 212 and the second part 213 are fixed.

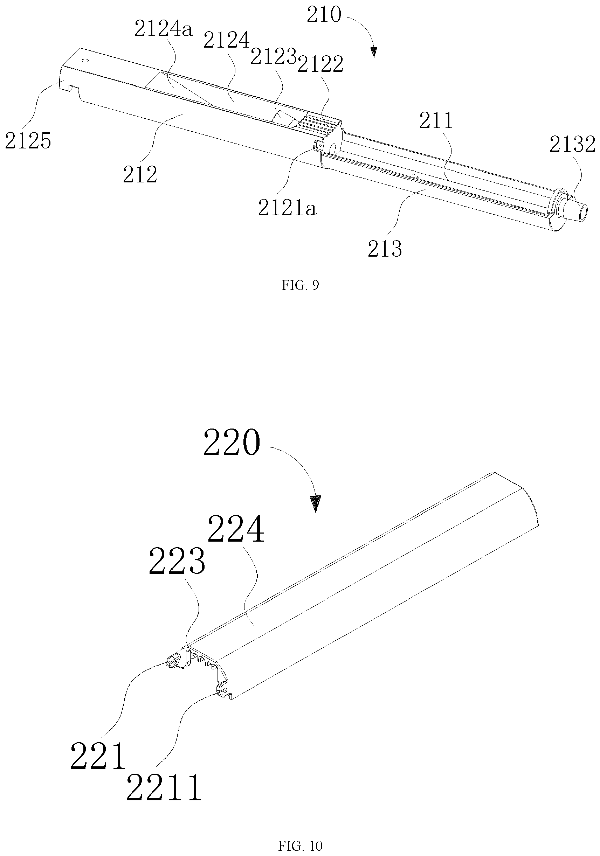

As shown in FIG. 9, the animal cabin 1 further includes a first joint 2123 and a second joint 2132 respectively disposed at both ends of the second part 213. The first joint 2123 is in communication with the second joint 2132 through the chamber 211. The first joint 2123 is configured to introduce a medium into the chamber 211, and the second joint 2132 is configured to discharge the existing medium in the chamber 211. The medium herein is air at different temperatures. Introducing air of different temperatures into the chamber 211 is mainly to provide different environmental temperatures for the animals to be scanned in the chamber 211. Certainly, in other embodiments, the medium can be various types of liquid substances. The selection of the medium needs to be changed according to different experiments, and is not limited herein. By arranging the first joint 2123 and the second joint 2132 in communication with the chamber 211 at both ends of the second part 213, it is convenient to supply different media to the chamber 211, thereby enabling better testing.

It should be noted that the first joint 2123 and the second joint 2132 are both air duct joints, which are configured to connect to an external temperature control system to provide air of different temperatures to the chamber 211. Certainly, in other embodiments, the first joint 2123 and the second joint 2132 can also be joints of other different devices, and can be configured to connect various different devices. For example, in the case that oxygen, anesthetic gas, etc. need to be input into the chamber 211, the first joint 2123 and the second joint 2132 are configured to connect the oxygen device or the anesthetic gas device, which mainly depends on the specific conditions of the experiment, and is not limited herein.

Further, the first part 212 is provided with an accommodating groove 2124 for accommodating an air duct. The accommodating groove 2124 has a first wall 2124a and a second wall 2124b. The first wall 2124a is located at an end of the accommodating groove 2124 away from the second part 213, and the second wall 2124b is located at an end of the accommodating groove 2124 adjacent to the second part 213. The first joint 2123 is disposed on the second wall 2124b and in communication with the chamber 211. When the air duct is placed in the accommodating groove 2124, one end of the air duct is connected to the external temperature control system, and the other end of the air duct is connected to the first joint 2123. Thereby, air flows with different temperatures are sent into the chamber 211 through the first joint 2123.

Specifically, the first wall 2124a is arranged at an angle to the axis of the second capsule 210. That is, the vertical distance from the first wall 2124a to the axis of the first part 212 gradually decreases in a direction toward the second part 213. By arranging the first wall 2124a at an angle to the axis of the second capsule 210, it not only facilitates the routing of the air duct, but also effectively avoids interference between the air duct and a volume coil at a later mounting position.

In some embodiments, the angle between the first wall 2124a and the axis of the second capsule 210 is in a range from 30° to 60°. That is, the angle between the first wall 2124a and the axis of the second capsule 210 may be 30°, 40°, 50°, 60°, etc., which is not limited herein. In the case that the angle between the first wall 2124a and the axis of the second capsule 210 is too small, it will not have the effect of avoiding interference between the air duct and the volume coil at the later mounting position. In the case that the angle between the first wall 2124a and the second capsule body 210 is too large, it will be difficult for the air duct to be placed smoothly in the accommodating groove 2124.

In an embodiment, the first joint 2123 is arranged at an angle to the axis of the second capsule 210, and the angle of the first joint 2123 is adapted to the angle of the first wall 2124a. The second wall 2124b is also tilted. The first joint 2123 is disposed on the second wall 2124b, and the first joint 2123 is perpendicular to the second wall 2124b, which facilitates the fixation of the first joint 2123 and the routing of the air duct.

Certainly, the first joint 2123 is not limited to being perpendicular to the second wall 2124b. In other embodiments, the first joint 2123 may be at an angle to the second wall 2124b, as long as the air duct can be routed through the first joint 2123.

Further, the first part 212 is also provided with a wire passing groove 2122 for routing various pipelines (such as experimental cables, wires, pipes, etc.) in the chamber 211. The wire passing groove 2122 is disposed on the first part 212 and is located outside the accommodating groove 2124, so as to avoid interference between the experimental cables, wires, pipes and other pipelines and the air duct, which may cause wiring failures.

In some embodiments, a plurality of wire passing grooves 2122 are provided and arranged side by side on the first part 212 to facilitate the routing of experimental cables, wires, pipes and other pipelines.

As shown in FIG. 10, the cover 220 is provided with wire ports 223. The wire ports 223 are located on a side of the cover 220 facing the chamber 211, and the wire ports 223 are disposed corresponding to the wire passing grooves 2122 on the first part 212. The wire ports 223 are configured to guide various external experimental cables, wires, pipes and other pipelines mounted in the wire passing grooves 2122 to the chamber 211 through the wire ports 223, so as to connect various components in the chamber 211. The pipelines are guided and arranged by the wire passing grooves 2122 and the wire ports 223. The wire passing grooves 2122 and wire ports 223 are adapted to guide the pipelines into the chamber 211, so that the wires will not interfere with each other and cause malfunctions, and the normal use of the wires will not be affected when the cover 220 is disassembled from the second part 213.

In some embodiments, the wire ports 223 are semicircular grooves that are recessed in a direction away from the accommodating groove 2124. Correspondingly, the wire passing grooves 2122 are semicircular grooves that are recessed toward the accommodating groove 2124. The combination of the wire passing groove 2122 and the wire port 223 can form a complete circular space, which is just convenient for routing pipeline with circular cross-sections and guiding the extension direction of the pipeline. Certainly, in other embodiments, the wire port 223 and the wire passing groove 2122 may also be square, triangular, and other shaped grooves, as long as the wire port 223 and the wire passing groove 2122 are combined to form a space that facilitates the pipeline to pass through, and are not specifically limited herein.

It should be understood that the wire ports 223 may be recesses provided only at the cover 220, or may be grooves with the same shape as the wire passing grooves 2122 but with the opposite recess direction. That is, the wire ports 223 may also extend from one end to the other end of the cover 220, so that the pipelines extend from the position of the wire ports 223 along the direction of the grooves in the cover 220 without being randomly scattered in the chamber 211, further enhancing the arrangement and guidance of pipelines. The specific arrangement manner of the wire ports 223 is not limited herein.

It should be noted that a plurality of wire ports 223 are provided, and the plurality of wire ports 223 are arranged side by side in the cover 220 corresponding to the wire passing grooves 2122. The number of wire ports 223 needs to correspond to the number of wire passing grooves 2122. Certainly, in other embodiments, only one wire port 223 and a corresponding wire passing groove 2122 may be provided. The number of the wire ports 223 and the number of the wire passing grooves 2122 are not specifically limited herein.

In some embodiments, the plurality of wire ports 223 and the plurality of wire passing grooves 2122 may both be in different sizes, only ensuring that the wire ports 223 and the corresponding wire passing grooves 2122 are in the same size. The purpose of this arrangement is to be able to match pipelines of different sizes in the animal cabin 1 at the same time. Certainly, in other embodiments, the wire ports 223 and the wire passing grooves 2122 may be in the same size, which is not limited herein.

Further, the animal cabin 1 further includes a locking member 230. The locking member 230 is inserted into the connection position of the cover 220 and the second part 213, so as to lock/unlock the second part 213 and the cover 220. Correspondingly, an end of the cover 220 adjacent to the first part 212 is provided with a positioning protrusion 221 extending toward the first part 212, and the first part 212 is provided with a groove 2121 adapted to the positioning protrusion 221. The positioning protrusion 221 extends into the groove 2121, which completes the positioning between the first part 212 and the cover 220. The positioning protrusion 221 and a groove wall of the groove 2121 are further provided with corresponding second connection holes 2211. The locking member 230 is provided with threads. When the locking member 230 is inserted through the second connection holes 2211 in the positioning protrusion 221 and the groove wall of the groove 2121 in sequence, the locking member 230 is rotated in a screwing direction into the second connection hole 2211 to lock the second part 213 and the cover 220, so that the second part 213 and the cover 220 are relatively fixed to each other. When the locking member 230 is rotated in an unscrewing direction out from the second connection hole 2211, and the locking member 230 is removed from the second connection hole 2211, the cover 220 can be detached from the second part 213, and the unlocking of the second part 213 and the cover 220 by the locking member 230 is completed.

In some embodiments, the locking member 230 is a screw; certainly, in other embodiments, the locking member 230 may be a bolt, a pin, or other structures, which are not limited herein.

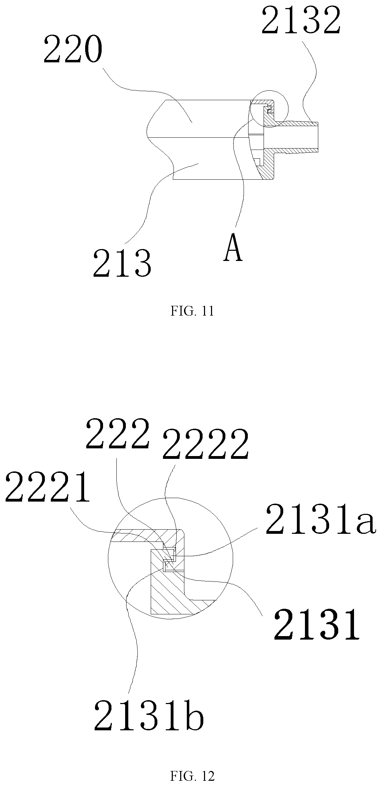

As shown in FIG. 11, an end of the cover 220 away from the first part 212 is provided with a first buckle 222. Correspondingly, an end of the second part 213 away from the first part 212 is provided with a second buckle 2131. The first buckle 222 is integrally formed with the cover 220, and the second buckle 2131 is integrally formed with the second part 213, which facilitates the processing of the first buckle 222 on the cover 220 and the processing of the second buckle 2131 on the second part 213. The first buckle 222 is configured to cooperate with the second buckle 2131 to achieve snapping and fixation between the cover 220 and the second part 213. Along the axis of the second capsule 210 and toward the direction of the first part 212, the first buckle 222 can be disengaged from the second buckle 2131 to realize the disassembly of the cover 220 from the second part 213. Along a direction perpendicular to the axis of the second capsule 210, and in a direction along the axis of the second capsule 210 and away from the first part 212, the first buckle 222 and the second buckle 2131 are fixed to realize the mounting and fixation of the cover 220 relative to the second part 213.

The first buckle 222 includes a first inserting portion 2221 and a first recessed portion 2222. The first inserting portion 2221 protrudes in a direction toward the first part 212, and the first recessed portion 2222 is recessed in a direction away from the first part 212. The second buckle 2131 includes a second inserting portion 2131a and a second recessed portion 2131b. The second inserting portion 2131a protrudes in a direction away from the first part 212, and the second recessed portion 2131b is recessed in a direction toward the first part 212. The first inserting portion 2221 extends into the second recessed portion 2131b for latching, and the second inserting portion 2131a extends into the first recessed portion 2222 for latching. In this way, along the axis of the second capsule 210 and in a direction towards the first part 212, the first inserting portion 2221 and the second recessed portion 2131b, and the second inserting portion 2131a and the first recessed portion 2222 can be disengaged from each other. In the direction perpendicular to the axis of the second capsule 210 and in the direction along the axis of the second capsule 210 and away from the first part 212, the first inserting portion 2221 and the second recessed portion 2131b, the second inserting portion 2131a and the first recessed portion 2222 are locked and fixed to each other.

It should be understood that, on the one hand, when the locking member 230 extends into the second connection hole 2211 to lock and fix the second part 213 with one end of the cover 220, the second part 213 and the other end of the cover 220 are also locked and fixed with each other, thereby avoiding the risk of the cover 220 being pushed open due to excessive pressure in the chamber 211. On the other hand, when the locking member 230 is loosened and taken out from the second connection hole 2211, the cover 220 can be easily pulled away in the direction toward the first part 212, thereby releasing the locking between the second part 213 and the other end of the cover 220, thereby completing the disassembly of the cover 220 conveniently and quickly.

In another embodiment of the present application, the first buckle 222 may further include two inserting portions extending in a direction away from the first part 212, and the two inserting portions are spaced apart, so that a recessed portion is formed between the two inserting portions. The second buckle 2131 may be configured such that the inserting portion extending toward the first part 212 is formed between two spaced recessed portions. In this way, the first buckle 222 and the second buckle 2131 can also be locked with each other. There are many ways of locking, and the specific locking manner between the cover 220 and the second part 213 is not limited herein.

Certainly, in other embodiments, the end of the cover 220 adjacent to the first part 212 and the second part 213 can be locked with each other, and the end of the cover 220 away from the first part 212 can be fixedly connected to the second part 213 through a locking member 230, which is not limited herein.

Further, the cover 220 at a side away from the second part 213 is a planar structure 224. The planar structure 224 extends from one end of the cover 220 to the other end of the cover 220 and is located at the top of the cover 220. The planar structure 224 is obtained by flattening the top of the original semi-cylindrical structure cover 220. The planar structure 224 is configured to prevent the animal cabin 1, when being pushed into a trapezoidal coil, from interference with the inner wall of the trapezoidal coil.

It should be noted that in magnetic resonance imaging device, the volume coil has a cylindrical hollow structure, and the inner wall of the volume coil is a smoothly cylindrical surface. When the animal to be scanned needs to be detected, the animal cabin 1 needs to be moved into a space surrounded by the volume coil, and then a magnetic resonance scan is performed on the animal. Therefore, the top of the cover 220 is partially cut off to prevent the top of the cover 220 from being in contact with and interfering with the volume coil when the animal cabin 1 is moved into the volume coil, thus affecting the normal experimental process. This can effectively reduce the probability of interference caused by subsequent animal support beds entering the volume coil.

Certainly, in other embodiments, the planar structure 224 may be in the shape of a curved surface, an inclined surface, a tapered surface, or other shapes. The purpose of arranging the planar structure 224 on the top of the cover 220 is only to reduce the height of the cover 220 and prevent the top of the cover 220 from colliding with the volume coil. Therefore, the planar structure 224 is not limited to a flat surface.

Further, an end the first part 212 away from the second part 213 is provided with a fixing portion 2125. The fixing portion 2125 may be integrally formed with the first part 212, or may be a separate part besides the first part 212, which is not limited herein. The fixing portion 2125 is connected to the first capsule 10, such that the second capsule 210 is connected to the first capsule 10.

In some embodiments, the fixing portion 2125 is configured in a snap-fit manner for snap-fitting with the first capsule 10. Certainly, in other embodiments, the fixing portion 2125 may be connected to the first capsule 10 through a threaded connection, which is not limited herein.

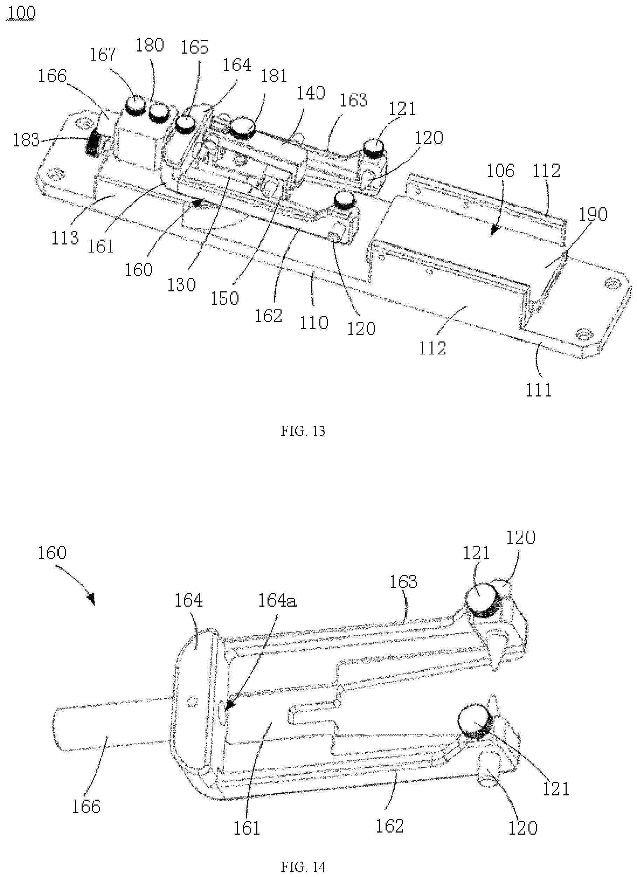

Referring to FIG. 13, in an embodiment, the animal cabin 1 further includes an animal fixation device 100. The animal fixation device 100 is mounted in the chamber 211 of the second capsule 210 and is configured to fix the animal to be scanned. The following description takes a mouse as an example for the animal, and the animal fixation device 100 is a mouse fixation device. Certainly, the animal may also be other animals with a similar body size to mice, such as rabbits, rats, etc., which are not specifically limited in the embodiments of the present application. Referring to FIGs. 14 to 16, the animal fixation device 100 includes a base 110, two ear positioning portion 120, a tooth positioning portion 130, a frontal bone positioning portion 140, a respiratory mask 150, and a support base 160.

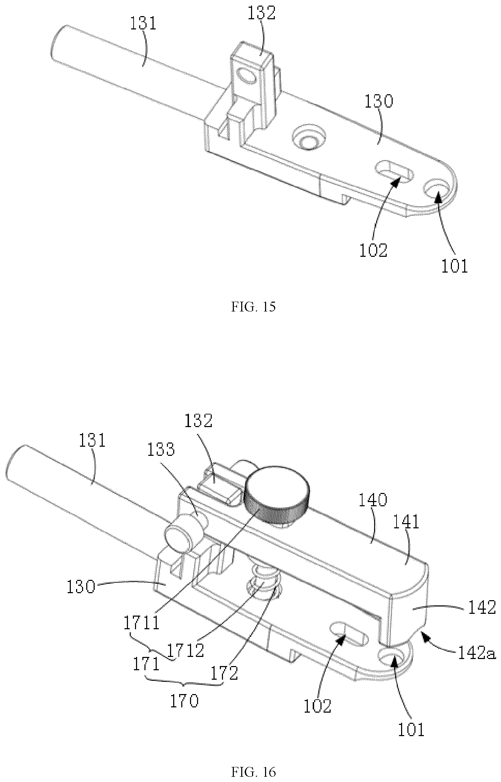

The support base 160 is mounted on the base 110. The two ear positioning portions 120 are spaced apart and mounted on the support base 160. The two ear positioning portions 120 are respectively configured to position two ears of the mouse. The tooth positioning portion 130 is mounted on the support base 160 and is located on the same side of the two ear positioning portions 120 (i.e., on the left side in FIG. 13). Referring to FIGs. 15 and 16, the tooth positioning portion 130 is provided with a tooth hole 101, which is used for the animal's teeth to bite. The frontal bone positioning portion 140 is located on a side of the tooth positioning portion 130 away from the base 110, and is configured to press the frontal bone of the animal toward the tooth positioning portion 130.

Specifically, as shown in FIG. 13, in some embodiments, the base 110 is in a shape of a rectangular plate as a whole. Certainly, the base 110 may also be in other shapes, such as a square or circular block, etc.

The two ear positioning portions 120 and the tooth positioning portion 130 are respectively mounted on the support base 160, so that they can be mounted on the base 110 indirectly. Specifically, as shown in FIGs. 13 and 14, in some embodiments, the support base 160 includes a support body 161, a first support arm 162, and a second support arm 163. The support body 161 may be plate-shaped or block-shaped. The support body 161 is mounted on the base 110. The first support arm 162 and the second support arm 163 are fixedly connected to the support body 161 respectively. The first support arm 162 and the second support arm 163 are disposed opposite to each other along the width direction of the base 110.

One ear positioning portion 120 is mounted on an end of the first support arm 162 away from the support body 161, and the other ear positioning portion 120 is mounted on an end of the second support arm 163 away from the support body 161, then the two ear positioning portions 120 are disposed opposite to each other along the width direction of the base 110. Each ear positioning portion 120 is provided with a first threaded connection member 121, and each ear positioning portion 120 can be fixed to the corresponding first support arm 162 or second support arm 163 through the corresponding first threaded connection member 121. As shown in FIG. 13, in some embodiments, the ear positioning portion 120 is an ear rod. The first support arm 162 and the second support arm 163 are respectively provided with an ear positioning hole (not shown). Each ear positioning portion 120 is mounted in one respective corresponding ear positioning hole, and the position of each ear positioning portion 120 can be adjusted along the corresponding ear positioning hole, so that the two ear positioning portions 120 can be close to or away from each other. When the position of the ear positioning portion 120 is adjusted along the corresponding ear positioning hole, it can be fixed on the corresponding first support arm 162 or second support arm 163 through the corresponding first threaded connection member 121.

As shown in FIGs. 13 and 14, the support base 160 further includes a first mounting protrusion 164. The first mounting protrusion 164 is disposed on the support body 161 and protrudes from the support body 161 in a direction away from the base 110 (i.e., upward in FIG. 13). As shown in FIG. 14, a first mounting hole 164a is provided on a surface of an end of the first mounting protrusion 164 adjacent to the two ear positioning portions 120. As shown in FIG. 15, the tooth mounting portion 130 includes a first mounting shaft 131 at an end thereof. The first mounting shaft 131 cooperates with the first mounting hole 164a, such that the tooth mounting portion 130 can be mounted on the first mounting protrusion 164. Referring to FIG. 13, the tooth mounting portion 130 and the first mounting protrusion 164 can be fixedly connected through a second threaded connection member 165. The tooth mounting portion 130 is located on a side of the first mounting protrusion 164 adjacent to the ear positioning portion 120.

As shown in FIGs. 13 and 16, in some embodiments, the frontal bone positioning portion 140 is mounted on the tooth positioning portion 130, thereby being indirectly mounted on the support base 160. The frontal bone positioning portion 140 is configured to press the frontal bone of the mouse toward the tooth positioning portion 130. For example, the frontal bone positioning portion 140 and the tooth positioning portion 130 may be fixedly connected by bolts. When to position the frontal bone of the mouse, the frontal bone positioning portion 140 and the tooth positioning portion 130 can be disconnected, the frontal bone of the mouse is placed above the tooth positioning portion 130, and then the frontal bone positioning portion 140 is placed above the tooth positioning portion 130 and fixedly connected to the tooth positioning portion 130, so that the frontal bone of the mouse can be tightly pressed between the frontal bone positioning portion 140 and the tooth positioning portion 130.

When to position the head of a mouse, the animal fixation device 100 can respectively position the ears, teeth and frontal bones of the mouse.

Specifically, the entire body of the mouse can be placed on the base 110, and the mouse's head is placed between the first support arm 162 and the second support arm 163, then the mouse's head is located between the two ear positioning portions 120. By adjusting the positions of the two ear positioning portions 120 along the corresponding ear positioning holes, the two ear positioning portions 120 can be inserted into the corresponding ears of the mouse, and the outer surfaces of the two ear positioning portions 120 can be clamped by the inner walls of the mouse's ear holes. In this case, the position of the ear positioning portion 120 is adjusted in place, and the ear positioning portion 120 can be fixed to the corresponding first support arm 162 or second support arm 163 through the corresponding first threaded connection member 121, so that the two ear positioning portions 120 can define the positions of the two ears of the mouse along the width direction of the base 110.

When positioning the mouse's teeth, the mouse's teeth need bite into the tooth hole 101. For example, the mouse's upper teeth can be inserted into the tooth hole 101 from top to bottom, and the mouse's lower teeth can be inserted into the tooth hole 101 from bottom to top. The frontal bone of the mouse is placed between the frontal bone positioning portion 140 and the tooth positioning portion 130, and the frontal bone positioning portion 140 is tightly pressed in a direction toward the tooth positioning portion 130 (i.e., downward in FIGs. 13 and 16), so that the frontal bone of the mouse is tightly clamped between the frontal bone positioning portion 140 and the tooth positioning portion 130. Since the mouse's teeth bite into the tooth hole 101, the frontal bone positioning portion 140 tightly presses the mouse's frontal bone downward, so that the mouth of the mouse can press against a surface of the tooth positioning portion 130, and the frontal bone positioning portion 140 and the tooth positioning portion 130 can limit the positions of the frontal bone portion and the mouth of the mouse in the thickness direction of the base 110. Furthermore, the frontal bone positioning portion 140 tightly presses the frontal bone of the mouse, so that the mouse's teeth are reliably pressed into the tooth hole 101, thereby further defining the position of the mouse's mouth along the radial direction of the tooth hole 101.

In some embodiments, a first direction is defined as the thickness direction of the base 110, a second direction is defined as the width direction of the base 110, and a third direction is defined as the length direction of the base 110.

It can be seen that a three-point (two ears and mouth) and one-side (frontal bone) positioning of the head of the mouse is achieved by the two ear positioning portions 120, the tooth positioning portion 130, and the frontal bone positioning portion 140. The positions of the frontal bone and the mouth of the mouse in a first direction (i.e., the thickness direction of the base 110), the positions of the two ear of the mouse in a second direction (i.e., the width direction of the base 110), and the position of the mouth of the mouse along the radial direction of the tooth hole 101 are limited, thereby reliably positioning the entire head of the mouse, and thus achieving a good fixation effect on the head of the mouse in an awake state.

It should be understood that the specific structure of the support base 160 is not limited to the structure described in the above embodiment. In other embodiments, the support base 160 may be in other structural forms. In other embodiments where the shape of the base 110 changes, the first direction, the second direction, and the third direction may be interchanged. For example, the third direction is the width direction, the second direction is the thickness direction, the first direction is the length direction, etc.

When the animal fixation device 100 of the above-mentioned animal cabin 1 is configured to position the animal's head, the two ear positioning portions 120 can limit the position of the two ears of the animal along the second direction (i.e., the width direction of the base 110). Since the animal's teeth bite into the tooth hole 101, the frontal bone positioning portion 140 tightly presses the animal's frontal bone toward the tooth positioning portion 130, so that the animal's mouth can press against the surface of the tooth positioning portion 130, and the frontal bone positioning portion 140 and the tooth positioning portion 130 can limit the positions of the animal's frontal bone and mouth along the first direction (i.e., the thickness direction of the base 110). Furthermore, the frontal bone positioning portion 140 tightly presses the animal's frontal bone, so that the animal's teeth are reliably pressed into the tooth hole 101, thereby further limiting the position of the animal's mouth along the radial direction of the tooth hole 101. It can be seen that the three-point and one-side positioning of the head of the animal is achieved by the two ear positioning portions 120, the tooth positioning portion 130, and the frontal bone positioning portion 140. The positions of the two ears of the animal's head along the second direction, the frontal bone and the mouth of the animal along the first direction, and the mouth along the radial direction of the tooth hole 101 are limited, so that the entire head of the animal is reliably positioned, and the good fixation effect on the head of the mouse in an awake state can be achieved.

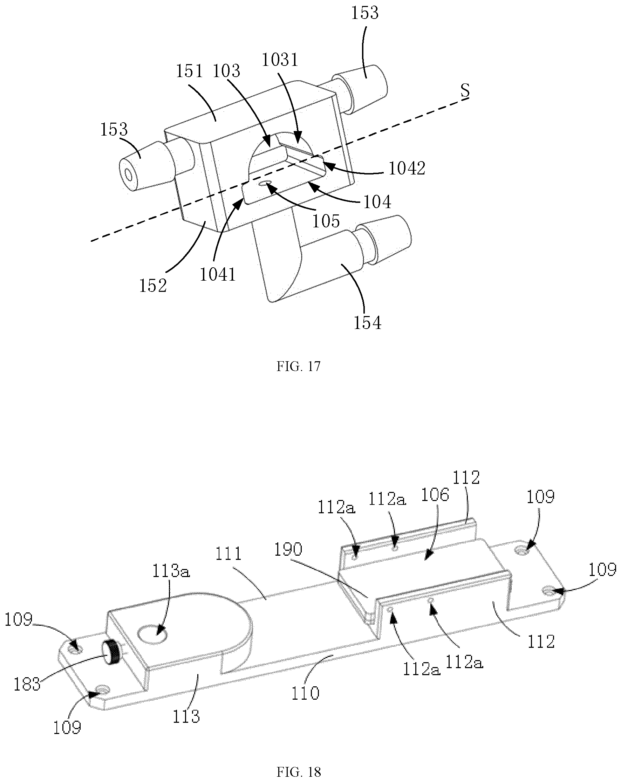

Referring to FIGs. 15 and 16, the tooth positioning portion 130 is provided with a ventilation hole 102. The ventilation hole 102 is located on a side of the tooth hole 101 away from the two ear positioning portions 120 (in FIG. 15, the ventilation hole 102 is located on the left side of the tooth hole 101). Referring to FIGs. 13 and 17, the respiratory mask 150 is at least partially located between the tooth positioning portion 130 and the frontal bone positioning portion 140. The respiratory mask 150 has an air chamber 103 and an air inlet channel (not shown). An opening 1031 of the air chamber 103 faces a direction of the two ear positioning portions 120 and is configured to allow the animal's nose to enter the air chamber 103. The air chamber 103 is in communication with the air inlet channel and the ventilation hole 102 respectively.

Referring to FIG. 17, in some embodiments, the respiratory mask 150 includes a mask body 151 and a mask mounting portion 152. The mask body 151 is located between the tooth positioning portion 130 and the frontal bone positioning portion 140. The air chamber 103 and the air inlet channel are respectively disposed on the mask body 151. The mask mounting portion 152 is fixed to the mask body 151 and is located on a side of the mask body 151 adjacent to the base 110. The mask mounting portion 152 is provided with a mounting groove 104 in communication with the air chamber 103. The tooth positioning portion 130 is inserted into the mounting groove 104 and adapted to the mounting groove 104. The mask mounting portion 152 is provided with an exhaust channel 105, and the position of the exhaust channel 105 corresponds to the position of the ventilation hole 102.

Specifically, as shown in FIG. 17, taking the dotted line S as a dividing line, the mask main body 151 is located above the dotted line S, and the mask mounting portion 152 is located below the dotted line S. The mask body 151 and the mask mounting portion 152 may be integrally formed to form an integral structure. In some embodiments, the shape outline formed by the combination of the two members is a cuboid. The air chamber 103 is in communication with the mounting groove 104.

It can be understood from FIG. 17 and FIG. 13 that the mounting groove 104 penetrates the mask mounting portion 152 along the third direction (i.e., the length direction of the base 110) and is a through groove. When mounting the respiratory mask 150, the respiratory mask 150 can be sleeved on an end of the tooth positioning portion 130, and the tooth positioning portion 130 is passively inserted into the mounting groove 104 and adapted to the mounting groove 104, so that the respiratory mask 150 can be integrally mounted on the tooth positioning portion 130, allowing easy mounting of the respiratory mask 150.

The opening 1031 of the air chamber 103 faces the direction of the two ear positioning portions 120, so that the mouse's nose can enter the air chamber 103 from the opening 1031. It should be understood that from the orientation shown in FIG. 13, the respiratory mask 150 is located on the left side of the tooth hole 101, and the opening 1031 of the air chamber 103 is to the right. Therefore, when placing the mouse, the mouse's nose is located at the left side of the mouse's teeth, so that the mouse's nose can enter the air chamber 103 from the opening 1031, and the teeth can bite into the tooth hole 101.

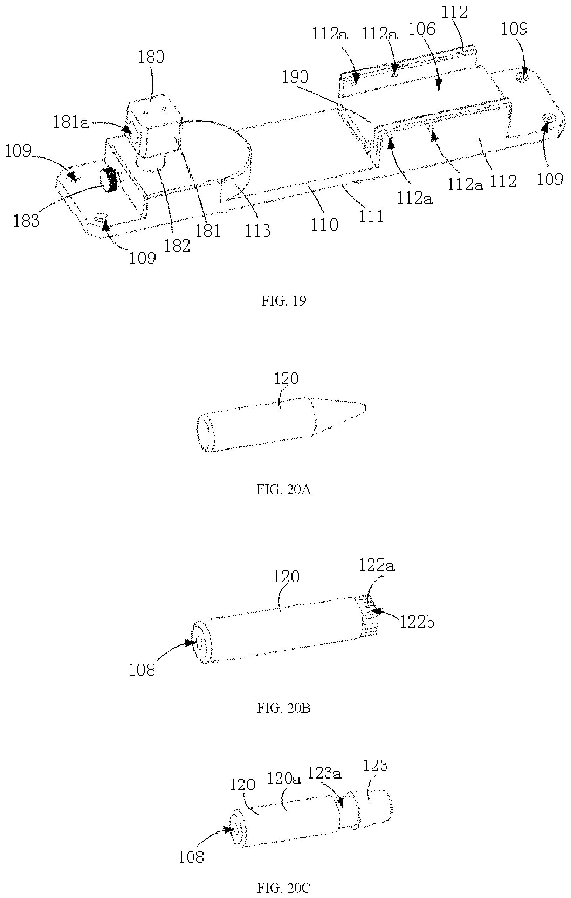

The mask body 151 is located between the tooth positioning portion 130 and the frontal bone positioning portion 140. It can be understood from FIG. 17 and FIG. 13 that when the respiratory mask 150 is sleeved on the tooth positioning portion 130, the inner wall of the air chamber 103 faces the surface of the tooth positioning portion 130, and the ventilation hole 102 is located in the tooth positioning portion 130, so that the air chamber 103 can be in communication with the ventilation hole 102. The mask mounting portion 152 is provided with the exhaust channel 105. As shown in FIG. 20, the exhaust channel 105 is located on the inner wall of the mounting groove 104. Since the position of the ventilation hole 102 corresponds to the position of the exhaust channel 105, the exhaust channel 105 is located below the ventilation hole 102, and the two members are in communication with each other.

Since the animal's nose can enter the air chamber 103, by inputting olfactory stimulating gas such as irritating gas or anesthetic gas into the air inlet channel, the olfactory stimulating gas can enter the air chamber 103 and be inhaled by the animal's nose. Since the air chamber 103 is in communication with the ventilation hole 102, and the ventilation hole 102 corresponds to the exhaust channel 105, the gas exhaled by the animal can be discharged from the air chamber 103 to the ventilation hole 102, and then discharged to the outside of the respiratory mask 150 through the exhaust channel 105. It can be seen that through the above-mentioned process of inputting olfactory stimulation gas and discharging the animal's exhaled gas, it is convenient to provide olfactory stimulation to the animal.

Referring to FIG. 17, in an embodiment, the mounting groove 104 has a first inner wall 1041 and a second inner wall 1042. The second inner wall 1042 is arranged opposite to the first inner wall 1041, and the second inner wall 1042 and the first inner wall 1041 are inclined to each other. The tooth positioning portion 130 is sandwiched between the first inner wall 1041 and the second inner wall 1042. Along the direction in which the tooth positioning portion 130 is inserted into the mounting groove 104, the distance between the second inner wall 1042 and the first inner wall 1041 gradually decreases.

Specifically, referring to FIGs. 13 and 17, it can be seen that in some embodiments, the first inner wall 1041 and the second inner wall 1042 are disposed opposite to each other along the second direction (i.e., the width direction of the base 110), so that when the tooth positioning portion 130 is inserted into the mounting groove 104, the tooth positioning portion 130 can be sandwiched between the first inner wall 1041 and the second inner wall 1042, so that the respiratory mask 150 is assembled to the tooth positioning portion 130.

In some embodiments, the second inner wall 1042 is an inclined surface, the first inner wall 1041 is an inclined surface, and the two surfaces are inclined to each other. Along the direction in which the tooth positioning portion 130 is inserted into the mounting groove 104 (i.e., to the right along the length direction of the base 110 in FIG. 13), the distance between the second inner wall 1042 and the first inner wall 1041 gradually decreases. That is, the mounting groove 104 gradually becomes narrower. Therefore, when the tooth positioning portion 130 is inserted into the mounting groove 104, it will become increasingly secure, so that the respiratory mask 150 and the tooth positioning portion 130 can be tightly assembled together.

Referring to FIG. 17, in an embodiment, the respiratory mask 150 further includes an air inlet pipe joint 153 and an exhaust pipe joint 154. The air inlet pipe joint 153 is disposed on the mask body 151 and in communication with the air inlet channel. The exhaust pipe joint 154 is disposed on the mask mounting portion 152 and in communication with the exhaust channel 105.

Specifically, as shown in FIG. 17, the air inlet pipe joint 153 is connected to the outer wall of the mask body 151, and two air inlet pipe joints 153 are provided. The two air inlet pipe joints 153 are arranged along the width direction of the base 110 and are located on different sides of the mask body 151. The exhaust pipe joint 154 is connected to the outer wall of the mask mounting portion 152 and is located on a side of the mask mounting portion 152 away from the mask body 151. One exhaust pipe joint 154 is provided.

Certainly, the number of the air inlet pipe joints 153 is not limited to two, and may be one or three. The arrangement of the air inlet pipe joints 153 is not limited to the arrangement in this embodiment. The number of the exhaust pipe joints 154 is not limited to one, but may be two or three.

The air inlet pipe joint 153 is configured to connect an air inlet pipe. The exhaust pipe joint 154 is configured to connect an exhaust pipe. When performing magnetic resonance imaging experimental studies on mice, it usually needs to place the animal fixation device 100 in an animal chamber (not shown) for experimental studies. The air inlet pipe is connected to air inlet pipe joint 153, and the air inlet pipe is extended outside the animal chamber, so that the olfactory stimulation gas can be input into the air inlet pipe from outside of the animal chamber. The olfactory stimulation gas can be inhaled by the mice after passing through the air inlet pipe, the air inlet channel, and the air chamber 103 in sequence, and thus facilitating the provision of the olfactory stimulation gas to the mice. Similarly, the exhaust pipe is connected to the exhaust pipe connector 154, and the exhaust pipe is extended outside the animal cabin, so that the gas exhaled by the mouse can be discharged outside the animal chamber through the air chamber 103, the ventilation hole 102, the exhaust channel, 105 and the exhaust pipe in sequence, thereby facilitating the discharge of the mouse's exhaled gas to the outside of the animal chamber.

Referring to FIG. 16, in an embodiment, the animal fixation device 100 further includes a second adjusting assembly 170. The second adjusting assembly 170 includes a limiting member 171 and a second elastic member 172. The limiting member 171 is adjustable and fixedly connected to the tooth positioning portion 130. The limiting member 171 is at least partially located on a side of the frontal bone positioning portion 140 away from the tooth positioning portion 130, and can adjust its position toward or away from the tooth positioning portion 130. The second elastic member 172 is located on a side of the tooth positioning portion 130 adjacent to the frontal bone positioning portion 140. The direction of the elastic force exerted by the second elastic member 172 on the frontal bone positioning portion 140 is away from the tooth positioning portion 130, such that an end of the frontal bone positioning portion 140 away from the tooth positioning portion 130 can abut against the limiting member 171.

Specifically, the limiting member 171 is a threaded connection member. The threaded connection member is, for example, a bolt or a screw. As shown in FIG. 16, it should be understood that the threaded connection member includes a connection head portion 1711 and a second threaded rod 1712. The connection head portion 1711 is fixed to the second threaded rod 1712 (may be integrally formed), and the outer diameter of the connection head portion 1711 is greater than the outer diameter of the second threaded rod 1712. In some embodiments, the connection head portion 1711 is located on a side of the frontal bone positioning portion 140 away from the tooth positioning portion 130. The second threaded rod 1712 extends through the frontal bone positioning portion 140 and the tooth positioning portion 130 in sequence, and is threadedly connected to the tooth positioning portion 130. Therefore, by rotating the limiting member 171 relative to the tooth positioning portion 130, the connection head portion 1711 can move close to or away from the tooth positioning portion 130.

In some embodiments, the second elastic member 172 is a coil spring. The coil spring is sleeved on the second threaded rod 1712 and is located between the tooth positioning portion 130 and the frontal bone positioning portion 140, and is in a compressed state. Therefore, an upper end of the coil spring abuts against the frontal bone positioning portion 140, and a lower end of the coil spring abuts against the tooth positioning portion 130. The coil spring can exert an elastic force on the frontal bone positioning portion 140 away from the tooth positioning portion 130, so that the end of the frontal bone positioning portion 140 away from the tooth positioning portion 130 can abut against the connection head portion 1711.

When to position the frontal bone of an animal, the limiting member 171 can be rotated in a certain direction (for example, clockwise or counterclockwise), so that the connection head portion 1711 can be moved in a direction away from the tooth positioning portion 130. When the connection head portion 1711 moves away from the tooth positioning portion 130, the elastic force of the second elastic member 172 simultaneously drives the frontal bone positioning portion 140 to move toward the connection head portion 1711, so that the frontal bone positioning portion 140 is kept abutting against the connection head portion 1711. That is, when the connection head portion 1711 moves in a direction away from the tooth positioning portion 130, the frontal bone positioning portion 140 also adjusts its position in the direction away from the tooth positioning portion 130, so that the distance between the frontal bone positioning portion 140 and the tooth positioning portion 130 can be increased, thereby facilitating the placement of the frontal bone of the mouse between the frontal bone positioning portion 140 and the tooth positioning portion 130. It should be understood that when the distance between the frontal bone positioning portion 140 and the tooth positioning portion 130 is large enough to facilitate the insertion of the mouse's frontal bone, the movement of the connection head portion 1711 can be stopped. When the connection head portion 1711 stops moving, since the elastic force of the second elastic member 172 can keep the frontal bone positioning portion 140 abutting against the connection head portion 1711, the position of the frontal bone positioning portion 140 can remain unchanged, thereby, even if without holding the frontal bone positioning portion 140 by hand, a stable distance between the frontal bone positioning portion 140 and the tooth positioning portion 130 can be maintained. In this case, it is very convenient to place the frontal bone of the mouse between the frontal bone positioning portion 140 and the tooth positioning portion 130.

Then, by rotating the limiting member 171 in a direction opposite to the direction described above, the connection head portion 1711 moves in a direction toward the tooth positioning portion 130, so that the connection head portion 1711 drives the frontal bone positioning portion 140 to overcome the elastic force of the second elastic member 172, causing the frontal bone positioning portion 140 to move in a direction toward the tooth positioning portion 130. Until the frontal bone positioning portion 140 tightly presses the frontal bone of the mouse, the rotation of the limiting member 171 is stopped. It can be seen that the frontal bone of the mouse can be tightly pressed by rotating the limiting member 171, which facilitates operation.

In this embodiment, the distance between the frontal bone positioning portion 140 and the tooth positioning portion 130 can be flexibly adjusted through the second adjusting assembly 170, so that the frontal bone of the animals can be pressed tightly for animals of different sizes, and it is easy to operate when positioning the frontal bone of the animals.

Referring to FIG. 16, in an embodiment, an end of the frontal bone positioning portion 140 is rotatably connected to the tooth positioning portion 130, and another end is configured to press the frontal bone of the animal. When the position of the limiting member 171 is adjusted in the direction away from the tooth positioning portion 130, the elastic force exerted by the second elastic member 172 on the frontal bone positioning portion 140 can drive the frontal bone positioning portion 140 to rotate, so that the frontal bone positioning portion 140 abuts against the limiting member 171.

Specifically, as shown in FIG. 16, the animal fixation device 100 further includes a first mounting post 132 and a rotating shaft 133. The first mounting post 132 is disposed on the tooth positioning portion 130 and protrudes from the tooth positioning portion 130 in a direction away from the base 110 (i.e., upward in FIG. 16). An end of the frontal bone positioning portion 140 is provided with a mounting recess (not labeled). The mounting recess is substantially "U" shaped. Two side walls of the mounting recess sandwich the first mounting post 132. The rotating shaft 133 extends through the two side walls of the mounting recess and the first mounting post 132 respectively, so that the end of the frontal bone positioning portion 140 can be rotatably connected to the tooth positioning portion 130 through the rotating shaft 133, and the angle of the frontal bone positioning portion 140 can be adjusted around the rotating shaft 133.

It can be seen from FIG. 13 and FIG. 16 that the axis direction of the rotating shaft 133 is along the width direction of the base 110, that is, the second direction. It can be understood that when the frontal bone positioning portion 140 rotates around the rotating shaft 133 relative to the tooth positioning portion 130, the position of the end of the frontal bone positioning portion 140 connected to the rotating shaft 133 remains unchanged, and the end of the frontal bone positioning portion 140 away from the rotating shaft 133 moves close to or away from the tooth positioning portion 130.

Referring FIGs. 13 and 16, when adjusting the position of the frontal bone positioning portion 140 through the second adjusting assembly 170 in the above embodiment, in the case that the positioning member 171 is adjusted in a direction away from the tooth positioning portion 130, the elastic force exerted by the second elastic member 172 on the frontal bone positioning portion 140 can drive the frontal bone positioning portion 140 to rotate counterclockwise around the rotating shaft 133, so that the distance between the end of the frontal bone positioning portion 140 away from the rotating shaft 133 and the tooth positioning portion 130 increases. It is convenient to place the frontal bone of the mouse between the frontal bone positioning portion 140 and the tooth positioning portion 130. In the case that the position of the limiting member 171 is adjusted in a direction close to the tooth positioning portion 130, the limiting member 171 drives the frontal bone positioning portion 140 to overcome the elastic force of the second elastic member 172, causing the frontal bone positioning portion 140 to rotate clockwise around the rotating shaft 133, so that the end of the frontal bone positioning portion 140 away from the rotating shaft 133 can tightly press the frontal bone of the mouse

Since one end of the frontal bone positioning portion 140 is rotatably connected to the tooth positioning portion 130, its position remains unchanged, while the position the other end of the frontal bone positioning portion 140 is adjusted under the action of the second adjusting assembly 170. Therefore, when the position of the other end of the frontal bone positioning portion 140 is set, both ends of the frontal bone positioning portion 140 can have reliable positions. For example, when the frontal bone positioning portion 140 tightly presses the frontal bone of a mouse, since both ends of the frontal bone positioning portion 140 have reliable positions, it can ensure that the frontal bone of the mouse is reliably positioned.

Referring to FIG. 16, in an embodiment, the end of the frontal bone positioning portion 140 for pressing the frontal bone of the mouse has an arc-shaped concave surface 142a. The concave surface 142a is configured to press the frontal bone of the mouse.

Specifically, since the end of the frontal bone positioning portion 140 for pressing the frontal bone of the mouse has an arc-shaped concave surface 142a, the concave surface 142a is easily adapted to the shape of the mouse's frontal bone, so that mouse's frontal bone can be pressed tightly and reliably, providing better comfort for the mouse.

Referring to FIG. 16, in an embodiment, the frontal bone positioning portion 140 includes a positioning body 141 and a bending portion 142. The bending portion 142 is fixed to an end of the positioning body 141. The bending portion 142 is bent from the end of the positioning body 141 in a direction toward the tooth positioning portion 130, and is configured to press the frontal bone of the mouse

As shown in FIG. 16, in some embodiments, a bending angle of the bending portion 142 relative to the positioning portion body 141 is approximately a right angle. The bending portion 142 is configured to press the frontal bone of the mouse. By providing the bending portion 142, and the bending portion 142 being bent in a direction toward the tooth positioning portion 130, the bending portion 142 is closer to the tooth positioning portion 130 than the positioning portion body 141, making it easier to press the frontal bone of the mouse. In some embodiments, the concave surface 142a is disposed on the bending portion 142.

Referring to FIG. 13, in an embodiment, the base 110 is provided with a body placement slot 106, and the body placement slot 106 is configured to accommodate the body of the mouse.

Specifically, as shown in FIG. 13, the base 110 includes a bottom plate 111 and two limiting plates 112. The two limiting plates 112 are fixed to the base 110 respectively (may be integrally formed). The two limiting plates 112 are disposed opposite to each other along the first direction (i.e., the width direction of the base 110), so that the body placement slot 106 is defined between the two limiting plates 112, which can be configured to accommodate the body of the mouse. As shown in FIG. 13, the two limiting plates 112 are spaced apart from the support base 160 along the third direction (i.e., the length direction of the base 110).

Referring to FIGs. 13 and 19, in an embodiment, the animal fixation device 100 further includes a mounting seat 180. The mounting seat 180 is mounted on the base 110. The support body 161 is mounted on the mounting seat 180. The mounting seat 180 can drive the support body 141 to adjust its angle relative to the base 110 around the first direction (the thickness direction of the base 110). When the angle of the support body 161 is adjusted around the first direction, the tooth positioning portion 130 and the frontal bone positioning portion 140 can be moved to the outside of the base 110 along the second direction (the width direction of the base 110). In some embodiments, the second direction is perpendicular to the first direction.

Specifically, referring to FIGs. 13, 14 and 19, the mounting seat 180 is provided with a second mounting hole 181a. An end of the support body 161 is provided with a second mounting shaft 166. The second mounting shaft 166 can be adapted to the second mounting hole 181a, and the second mounting shaft 166 can be fixedly connected to the mounting seat 180 through a third threaded connection member 167, so that the support body 161 can be mounted on the mounting seat 180.

As shown in FIGs. 18 and 19, in some embodiments, the base 110 further includes a second mounting protrusion 113. The second mounting protrusion 113 can be integrally formed with the bottom plate 111. The second mounting protrusion 113 protrudes upward from the surface of the bottom plate 111. An end of the second mounting protrusion 113 away from the bottom plate 111 is provided with a third mounting hole 113a.

The mounting seat 180 includes a mounting body 181 and a third mounting shaft 182. The axial direction of the third mounting shaft 182 is along the second direction. One end of the third mounting shaft 182 is adapted to the third mounting hole 113a, and the other end is fixed to the mounting body 181. The mounting seat 180 further includes a fourth threaded connection member 183. The third mounting shaft 182 can be fixedly connected to the second mounting protrusion 113 through a fourth threaded connection member 183. A side wall of the third mounting hole 113a is provided with a through hole (not shown). The fourth threaded connection member 183 extends through the through hole and abuts against the third mounting shaft 182, thereby fixedly connecting the third mounting shaft 182 to the second mounting protrusion 113.

When to adjust the angle of the mounting seat 180, the fourth threaded connection member 183 can be loosened, and the mounting seat 180 can rotate around the axis of the third mounting shaft 182, so that the angle of the mounting seat 180 can be adjusted around the second direction, thereby adjusting the angle of the support body 161 along with the mounting seat 180 around the second direction.

When the angle of the support body 161 is adjusted around the first direction, the entire support base 160, the two ear positioning portions 120, the frontal bone positioning portion 140, and the tooth positioning portion 130 can be driven to rotate around the first direction. In some embodiments, when the angle of the support body 161 is adjusted around the first direction, the frontal bone positioning portion 140 and the tooth positioning portion 130 can be driven to move to the outside of the base 110 along the second direction (the width direction of the base 110). Therefore, when positioning the mouse's head, the mouse's head can be located outside the base 110 in the second direction. An operator located outside the base 110 in the second direction can position the ear, mouth, frontal bone, etc. of the mouse, making it less likely to be obstructed by the base 110 and facilitating the operation. When the mouse's head is positioned, the angle of the support body 161 can be adjusted in the second direction by rotating the mounting seat 180, so that the mouse's head is rotated to the base 110, and the mouse's body is rotated to the base 110 and placed in the body placement slot 106, and then the mounting seat 180 is fixed to the base 110.

FIG. 20A is a schematic structural view of the ear positioning portion 120 according to an embodiment.

FIG. 20B is a schematic structural view of the ear positioning portion 120 according to another embodiment. In some embodiments, the outer peripheral surface of an end of the ear positioning portion 120 for inserting into the ear hole of the mouse is provided with a plurality of teeth 122a and tooth grooves 122b arranged alternately in a circumferential direction, so that the outer peripheral surface of the end of the ear positioning portion 120 for inserting into the ear hole of the mouse forms a toothed surface, which can increase the contact area with the inner wall of the ear hole of the mouse, making the head of the mouse more securely fixed.

In other embodiments, the outer peripheral surface of the end of the ear positioning portion 120 for inserting into the ear hole of the mouse is not limited to the toothed surface, and may be other types of rough surfaces, such as a surface with anti-skid texture, a surface with anti-skid particles or anti-skid protrusions, etc. It should be understood that the rough surface is configured to increase friction between the ear positioning portion 120 and the inner wall of the mouse's ear hole. By providing any of these types of rough surfaces, the friction between the ear positioning portion 120 and the inner wall of the mouse's ear hole can be increased, thereby enabling a more secure fit between the ear positioning portion 120 and the mouse's ear hole, and facilitating a more secure fixation of the mouse's head.

FIG. 20C is a schematic structural view of the ear positioning portion 120 according to yet another embodiment. In some embodiments, the ear positioning portion 120 includes a body portion 120a and a tapering segment 123. The tapering segment 123 is fixed to and integrally formed with the body portion 120a=. An annular groove 123a is provided at the connection between the body portion 120a and the tapering segment 123. The tapering segment 123 is configured to be inserted into the mouse's ear hole. Along the direction of inserting into the mouse's ear hole, the outer diameter of the tapering segment 123 gradually decreases. In an embodiment, an earmuff is disposed on the ear rod. The earmuff can be made of a material with a high friction coefficient. Specifically, the earmuff (not shown) can be sleeved on the tapering segment 123 in a direction in which the outer diameter of the tapering segment 123 gradually increases (i.e., from right to left in FIG. 20c), so that an open end of the earmuff can be snapped into the annular groove 123a. Moreover, since the outer diameter of the tapering segment 123 gradually increases, it is advantageous for the earmuff to fit firmly on the tapering segment 123. Furthermore, since the tapering segment 123 is sleeved with the earmuff, the friction with the animal's ears can be increased, and the animal's ears are not easily damaged, and can be adapted to animals of different sizes.

Referring to FIG. 20B and FIG. 20C, in an embodiment, the ear positioning portion 120 is provided with an auditory stimulation input hole 108, and the auditory stimulation input hole 108 is configured to be in communication with the animal's ear hole.

Specifically, when the ear positioning portion 120 is inserted into the mouse's ear, the auditory stimulation input hole 108 can be in communication with the mouse's ear hole, thereby facilitating the input of auditory stimulation to the mouse's ear.

Referring to FIGs. 18 and 19, the base 110 is provided with a plurality of base mounting holes 109. In some embodiments, four base mounting holes 109 are provided, which are respectively distributed at four corners of the base 110. Threaded connecting members (not shown) can be configured to cooperate with the base mounting holes 109 to secure the base 110 to the mounting plate in the animal chamber.

Referring to FIGs. 18 and 19, the limiting plate 112 is provided with a plurality of coil mounting holes 112a. In some embodiments, each limiting plate 112 is provided with two coil mounting holes 112a. In experimental studies of magnetic resonance imaging performed on animals, the radio frequency coil can be sleeved on the mouse fixation device 100, and mounted on the limiting plate 112 through cooperation between the coil mounting holes 112a and threaded connecting members (not shown).

Referring to FIG. 13, in an embodiment, the mouse fixation device 100 further includes a height adjustment pad 190. The height adjustment pad 190 is disposed on the base 110 and located in the body placement slot 106 for raising the position of the mouse's body.

Specifically, the height adjustment pads 190 with various thicknesses such as 2 mm, 4 mm, etc. can be provided. For mice of different body sizes, different height adjustment pads 190 can be used. Certainly, a plurality of height adjustment pads 190 may be used together. For example, two height adjustment pads with a thickness of 2 mm are used at the same time to form a height adjustment pad with a thickness of 4 mm. The plurality of height adjustment pads of different thicknesses can also be used in combination to form the desired height to accommodate mice of different sizes.

An embodiment of the present application further provides an animal imaging device (not shown). The animal imaging device includes the above-mentioned animal cabin 1 and a scanning device (not shown). The scanning device is configured with a scanning chamber that can accommodate the animal cabin 1. The animal cabin 1 can extend into the scanning chamber, and the scanning device is configured to scan and image the animals in the animal cabin 1. This animal imaging device also has the advantages of the animal cabin 1 described above.

In an embodiment, the animal imaging device further includes an animal support bed (not shown). The first capsule 10 is connected to the animal support bed.

In an embodiment, the animal imaging device is a magnetic resonance imaging device.

In an embodiment, the animal imaging device is a preclinical magnetic resonance imaging device. The scanning device is a magnetic resonance device. Certainly, the animal cabin 1 in the embodiment of the present application can also be used in other animal imaging device. The scanning device may also be other scanning devices. For example, a positron emission tomography (PET) and combined scanning device (such as PET-MR) and the like.

The above-mentioned embodiments do not constitute a limitation on the protection scope of the technical solution. Any modifications, equivalent replacements and improvements made within the spirit and principles of the above-mentioned embodiments shall be included within the protection scope of this technical solution.

The foregoing descriptions are merely specific embodiments of the present disclosure, but are not intended to limit the protection scope of the present disclosure. Any variation or replacement readily figured out by a person skilled in the art within the technical scope disclosed in the present disclosure shall all fall within the protection scope of the present disclosure.

## Claims

1. An animal cabin, comprising a first capsule (10) and a second capsule (210), and the first capsule (10) being detachably connected to the second capsule (210).

2. The animal cabin according to claim 1, further comprising a first adjusting assembly (30), wherein the first capsule (10) and the second capsule (210) are connected through the first adjusting assembly (30);
the animal cabin further comprises a rotating portion (40) located between the first capsule (10) and the second capsule (210); and under an adjustment of the first adjusting assembly (30), the second capsule (210) is capable of rotating around the rotating portion (40) relative to the first capsule (10) to adjust an angle between the first capsule (10) and the second capsule (210).

3. The animal cabin according to claim 2, wherein the rotating portion (40) comprises a rotating convex portion (41) and a rotating concave portion (42);
the rotating convex portion (41) is located at an end of the first capsule (10) adjacent to the second capsule (210), the rotating concave portion (42) is located at an end of the second capsule (210) adjacent to the first capsule (10), and the rotating convex portion (41) concave-convex fits with the rotating concave portion (42); or
the rotating concave portion (42) is located at an end of the first capsule (10) adjacent to the second capsule (210), the rotating convex portion (41) is located at an end of the second capsule (210) adjacent to the first capsule (10), and the rotating convex portion (41) concave-convex fits with the rotating concave portion (42).

4. The animal cabin according to claim 3, wherein the rotating convex portion (41) has a first arc surface (411) thereon, and the rotating concave portion (42) has a second arc surface (421) therein, and the first arc surface (411) and the second arc surface (421) are attached to each other in accordance with the concave-convex fit of the rotating convex portion (41) and the rotating concave portion (42).

5. The animal cabin according to claim 2, wherein the first adjusting assembly (30) comprises an adjusting rod (31), the second capsule (210) is provided with a first connection hole (21) extending through the second capsule (210), the first capsule (10) is provided with an adjustment hole (11), an end of the adjusting rod (31) extends through the first connection hole (21) and is capable of cooperating with the adjustment hole (11) to adjust an angle of the second capsule (210) relative to the first capsule (10).

6. The animal cabin according to claim 5, wherein the adjusting rod (31) comprises a first threaded rod (311) with a head (312), and the head abuts against the second capsule (210), the adjustment hole (11) is a threaded hole, and the first threaded rod (311) is in threaded fit with the adjustment hole (11).

7. The animal cabin according to claim 6, wherein a side wall of the first capsule (10) adjacent to the second capsule (210) is provided with an abutting portion (14), a side wall of the second capsule (210) adjacent to first capsule (10) is provided with a receiving groove (24) configured to accommodate the abutting portion (14), and when the first capsule (10) is connected to the second capsule (210), the abutting portion (14) abuts against a groove wall of the receiving groove (24).

8. The animal cabin according to claim 6, wherein the second capsule (210) is provided with a mounting hole (22) at a side towards the first capsule (10), the mounting hole (22) is in communication with the first connection hole (21);
an elastic unit (32) is provided in the mounting hole (22), and the elastic unit (32) is capable of elastically abutting against the first capsule (10) as adjustment of the adjusting rod (31).

9. The animal cabin according to claim 1, further comprising a cover (220), wherein the second capsule (210) is provided with a chamber (211), the cover (220) is detachably connected to the second capsule (210), and the cover (220) is capable of covering and being fixed on the second capsule (210).

10. The animal cabin according to claim 9, further comprising a locking member (230), wherein the locking member (230) is disposed on the cover (220) and an end of the second capsule (210), the locking member (230) is capable of locking the cover (220) and the second capsule (210), thereby locking and fixing the cover body (220) along an axis direction of the second capsule (210), and the cover body (220) and another end of the second capsule body (210) are engaged with each other.

11. The animal cabin according to claim 10, wherein the second capsule (210) comprises a first part (212) and a second part (213) arranged sequentially in the axis direction of the second capsule (210), and the cover (220) covers and detachably connected to the second part (213);
an end of the cover (220) adjacent to the first part (212) is provided with a positioning protrusion (221) extending toward the first part (212), the first part (212) is provided with a groove (2121) adapted to the positioning protrusion (221), the positioning protrusion (221) extends into the groove (2121), the groove (2121) has a groove wall (2121a), and the locking member (230) is inserted through the positioning protrusion (221) and the groove wall (2121a) to lock the second capsule (210) and the cover (220).

12. The animal cabin according to claim 10, wherein the second capsule (210) comprises a first part (212) and a second part (213) arranged sequentially in the axis direction of the second capsule (210), and the cover (220) covers and detachably connected to the second part (213);
an end of the cover (220) away from the first part (212) is provided with a first buckle (222), an end of the second part (213) away from the first part (212) is provided with a second buckle (2131), the first buckle (222) and the second buckle (2131) are capable of being locked with each other;
along the axis direction of the second capsule (210), the first buckle (222) and the second buckle (2131) are capable of separating from each other; and
along a direction perpendicular to the axis direction of the second capsule (210), the first buckle (222) and the second buckle (2131) are locked with each other.

13. The animal cabin according to claim 11 or 12, further comprising a first joint (2123) and a second joint (2132) respectively disposed at both ends of the second part (213), wherein the first joint (2123) and the second joint (2132) are in communication with the chamber (211).

14. The animal cabin according to claim 11 or 12, wherein a top of the cover (220) away from the second part (213) is a planar structure (224).

15. The animal cabin according to claim 1, further comprising an animal fixation device (100) mounted in the second capsule (210) and configured to fix an animal to be scanned.

16. The animal cabin according to claim 15, wherein the animal fixation device (100) comprises:
a support base (160);
a tooth positioning portion (130), mounted on the support base (160) and configured to position teeth of an animal; and
a respiratory mask (150), configured to provide an air channel for the animal, and the respiratory mask (150) being fixed on the tooth positioning portion (130).

17. The animal cabin according to claim 16, wherein the respiratory mask (150) comprises a mask mounting portion (152) and an air chamber (103), the mask mounting portion (152) is provided with a mounting groove (104) in communication with the air chamber (103), and the tooth positioning portion (130) is inserted into the mounting groove (104).

18. The animal cabin according to claim 17, wherein the mounting groove (104) has a first inner wall (1041) and a second inner wall (1042) opposite to each other, the tooth positioning portion (130) is sandwiched between the first inner wall (1041) and the second inner wall (1042), and along a direction in which the tooth positioning portion (130) is inserted into the mounting groove (104), a distance between the first inner wall (1041) and the second inner wall (1042) gradually decreases.

19. The animal cabin according to claim 17, wherein the tooth positioning portion (130) is provided with a tooth hole (101), the tooth positioning portion (130) is provided with a ventilation hole (102), the mask mounting portion (152) is provided with an exhaust channel (105), and a position of the exhaust channel (105) corresponds to a position of the ventilation hole (102).

20. The animal cabin according to claim 16, wherein the animal fixation device (100) further comprises a frontal bone positioning portion (140) mounted on the support base (160), and the frontal bone positioning portion (140) is configured to press the frontal bone of the animal in a direction toward the tooth positioning portion (130).

21. The animal cabin according to claim 20, wherein the animal fixation device (100) further comprises a second adjusting assembly (170), the second adjusting assembly (170) comprises:
a limiting member (171), adjustably connected to the tooth positioning portion (130) and is capable of adjusting a position thereof in a direction toward or away from the tooth positioning portion (130); and
a second elastic member (172), configured to exert an elastic force on the frontal bone positioning portion (140) in a direction away from the tooth positioning portion (130), such that an end of the frontal bone positioning portion (140) away from the tooth positioning portion (130) abuts against limiting member (171).

22. The animal cabin according to claim 21, wherein the limiting member (171) is a threaded connection member and comprises a connection head portion (1711) and a second threaded rod (1712) fixed to the connection head portion (1711), the connection head portion (1711) is located on a side of the frontal bone positioning portion (140) away from the tooth positioning portion (130), the second threaded rod (1712) extends through the frontal bone positioning portion (140) and is threadedly connected to the tooth positioning portion (130), the second elastic member (172) is sleeved on the second threaded rod (1712) and is located between the frontal bone positioning portion (140) and the tooth positioning portion (130); an end of the frontal bone positioning portion (140) is rotatably connected to the tooth positioning portion (130), and when the threaded connection member rotates, the threaded connection member or the second elastic member (172) is capable of driving the frontal bone positioning portion (140) to rotate relative to the tooth positioning portion (130).

23. The animal cabin according to claim 16, wherein the animal fixation device (100) comprises the support base (160) and two ear rods opposite to each other, the ear rods are mounted on the support base (160), each ear rod is provided with an auditory stimulation input hole (108); and an end of each ear rod has a rough surface or an earmuff is provided on each ear rod.

24. An animal imaging device, comprising the animal cabin according to any one of claims 1 to 23 and a scanning device, wherein the scanning device has a scanning chamber, the animal cabin is capable of extending into the scanning chamber, and the scanning device is configured to scan and form an image of an animal in the animal cabin.
